# FASCICULE DE BREVET EUROPEEN

(11) **EP 0 529 033 B1**
(45) Date de publication et mention de la délivrance du brevet: **30.08.2006**
(21) Numéro de dépôt: 92905817.0
(22) Date de dépôt: 07.02.1992
(51) Int. Cl.: C12N 15/63, A61K 39/395, G01N 33/577, C12Q 1/68

(54) **SEQUENCES NUCLEOTIDIQUES CODANT POUR DES REGIONS VARIABLES DE CHAINES ALPHA DES RECEPTEURS DES LYMPHOCYTES HUMAINS AINSI QUE LEURS APPLICATIONS**
NUKLEOTIDSEQUENZEN, DIE FÜR DIE VERÄNDERLICHEN BEREICHE DER ALPHA-KETTEN MENSCHLICHER T-ZELL-REZEPTOREN KODIEREN SOWIE IHRE VERWENDUNGEN
NUCLEOTIDE SEQUENCES CODING FOR ALPHA CHAIN VARIABLE REGIONS IN HUMAN LYMPHOCYTE RECEPTORS AND APPLICATIONS THEREOF

(30) Priorité: 08.02.1991 FR 9101487; 12.04.1991 FR 9104527
(43) Date de publication de la demande: 03.03.1993
(60) Demande divisionnaire: 06009929.8
(73) Titulaire: Aventis Pharma S.A., 92160 Antony (FR)
(72) Inventeur: HERCEND, Thierry, F-94130 Nogent-sur-Marne (FR); TRIEBEL, Frédéric, F-92200 Neuilly-sur-Seine (FR); ROMAN-ROMAN, Sergio, F-75014 Paris (FR); FERRADINI, Laurent, F-75014 Paris (FR)
(86) Numéro de dépôt international: PCT/FR1992/000111
(87) Numéro de publication internationale: WO 1992/013949

(56) Documents cités:
- EP-A- 0 261 671
- WO-A-87/03600
- WO-A-90/06758
- US-A- 4 874 845
- EUROPEAN JOURNAL OF IMMUNOLOGY vol. 21, no. 4, 9 Avril 1991, VCH VERLAGSGESELLSCHAFT, DEUTSCHLAND pages 927 - 933; Roman-Roman, S. et al.: 'Studies on the human T cell receptor alpha/beta variable region genes. I. Identification of 7 additional Valpha subfamilies and 14 J alpha gene segments.'
- EUROPEAN JOURNAL OF IMMUNOLOGY vol. 17, 1987, VCH VERLAGSGESELLSCHAFT, DEUTSCHLAND pages 375 - 383; Kimura, N. et al.: 'Sequences and repertoire of the human T cell receptor alpha and beta chain variable region genes in thymocytes.'
- THE JOURNAL OF EXPERIMENTAL MEDECINE vol. 164, no. 1, 1 Juillet 1986, THE ROCKEFELLER UNIV. PRESS pages 90 - 103; Yoshika, Y. et al.: 'Sequences and repertoire of human T cell receptor alpha chain variable region genes in mature T lymphocytes.'
- THE JOURNAL OF EXPERIMENTAL MEDECINE vol. 164, no. 6, 1 Décembre 1986, THE ROCKEFELLER UNIV. PRESS pages 1940 - 1957; Calman, A.F. et al.: 'Expression of T cell receptor genes in human B cells.'
- EMBO JOURNAL. vol. 7, no. 6, 1988, EYNSHAM, OXFORD GB pages 1661 - 1668; Baer, R. et al.: 'Complex rearrangements within the human Jdelta-Cdelta /Jalpah-Calpha locus and aberrant recombination between Jalpha segments.'
- PROCEEDINGS OF THE NATIONAL ACADEMY OF SCIENCES OF USA. vol. 86, 1989, WASHINGTON US pages 602 - 606; Russo. G. et al.: 'Molecular analysis of a t(14;14) translocation in leukemie T-cells of an ataxia telangiectasia patient.'

## Description

La présente invention concerne une nouvelle, séquence nucléotidique codant pour une région variable de chaîne α des récepteurs des cellules T, le segment peptidique correspondante et les applications diagnostiques et thérapeutiques.

Il est connu que les récepteurs reconnaissant les antigènes à la surface des lymphocytes T matures (désignés ci-après récepteurs des cellules T) possèdent une structure ayant une certaine analogie avec celles des immunoglobulines. Ainsi, ils comprennent des structures hétérodimériques comportant des chaînes glycoprotéiques α et β ou des chaînes glycoprotéiques γ et δ (voir Meuer et al. (1), Moingeon et al. (2), Brenner et al. (3), Bank et al. (4)).

Le répertoire des récepteurs des cellules T doit pouvoir faire face à l'immense diversité des déterminants antigéniques. Ceci est obtenu par recombinaison génétique des différents segments discontinus des gènes qui codent pour les différentes régions structurales des récepteurs des cellules T. Ainsi, les gènes comprennent des segments V (segments variables), éventuellement des segments D (segments de diversité), des segments J (segments de jonction) et des segments C (segments constants). Pendant la différenciation des cellules T, des gènes spécifiques sont créés par recombinaison des segments V, D et J pour les locus β et δ et des segments V et J pour les locus α et γ. Ces combinaisons spécifiques ainsi que l'appariement des deux chaînes créent la diversité combinatoire. Cette diversité est fortement amplifiée par deux mécanismes supplémentaires, à savoir la réunion imprécise des segments V-D-J ou V-J et l'addition de nucléotides correspondant à la région N (Davis et al. (5)).

On connaît déjà un certain nombre de segments géniques V. Ces segments ont été groupés en sous-familles en fonction de la similarité des séquences. Par définition, les segments qui présentent plus de 75 % de similarité dans la séquence nucléotidique ont été considérés comme des membres de la même sous famille (Crews et al. 6)). Les segments géniques Vα connus ont ainsi été classés en 22 sous familles dont 14 avec un seul membre (voir Concannon et al. (7), Kimura et al. (8), Wilson et al. (9)).

Par ailleurs, environ 60 segments géniques Jα ont été décrits (9).

On a par ailleurs décrit récemment dans WO 90/06758 des anticorps monoclonaux dirigés contre des segments spécifiques des parties variables des récepteurs des cellules T, notamment des chaînes *β* et δ. Ces anticorps monoclonaux sont utiles non seulement comme outils de diagnostic mais également comme outils thérapeutiques, par exemple vis-à-vis de l'arthrite rhumatoïde.

On a également décrit l'utilisation de peptides synthétiques correspondant à des régions variables des chaînes α ou β dans le traitement des maladies auto-immunes (23 et 24).

Il est connu par ailleurs qu'il existe des variations d'un individu à un autre dans l'expression des différents segments variables du récepteur T chez l'homme (27 et 28).

La présente invention vise à enrichir le répertoire des segments géniques codant pour des régions variables de chaînes a des récepteurs des cellules T en fournissant. nouveau segment génique Vα.

La présente invention a ainsi pour objet une séquence nucléotidique codant pour une région variable de chaîne α des récepteurs des lymphocytes T humains, correspondant à l'ADNc comprenant la séquence nucléotidique

du segment Vα correspondant à 20 SEQ ID N° 1 et les séquences qui en diffèrent par un ou plusieurs nucléotides.

La présente invention a également plus particulièrement pour objet :
- Une séquence nucléotidique codant pour une région variable de chaîne α des récepteurs des lympho-cytes T humains correspondant à l'ADNc correspondant à tout ou partie de la séquence nucléotidique du segment Vα correspondant à la séquence
   1 à 200 de SEQ ID N° 1

Par l'expression "séquences nucléotidiques correspondant à des ADNc correspondant à tout ou partie des séquences nucléotidiques", on désigne aussi bien les séquences complètes que des fragments de ces séquences y compris des fragments courts (oligonucléotides) qui trouvent des applications en tant que sondes (comportant généralement au moins 10 nucléotides) ou en tant qu'amorce (comportant généralement au moins 15 nucléotides). D'une manière générale, l'invention englobe l'ensemble des nouveaux oligonucléotides qui sont des fragments de la séquence Vα selon l'invention.

Quant aux séquences qui diffèrent par un ou deux nucléotides, elles correspondent à des variations que l'on a observé expérimentalement lors de la détermination de la séquence nucléotidique de plusieurs ADNc.

La présente invention a également pour objet les peptides codés par des séquences nucléotidiques selon l'invention ainsi que les allèles et les dérivés de ceux-ci qui possèdent la même fonction.

D'une manière générale, la présente invention englobe les peptides constitués par ou comprenant une séquence peptidique codée par les séquences nucléotidiques selon l'invention ainsi que les fragments de ces peptides. Elle englobe également les peptides qui diffèrent de ceux-ci d'un ou plusieurs amino-acides et qui possèdent la même fonction. Ces peptides peuvent correspondre à des modifications telles que celles connues avec les mutéines ou à des variations allèliques. Il a été en effet montré en particulier que certains segments géniques codant pour des régions variables de chaînes du récepteur T chez l'homme étaient soumis à un phénomène de polymorphisme génétique appelé variation allèlique (25). La présente invention englobe les peptides provenant de ce phénomène.

Le séquence nucléotidique selon l'invention a été obtenue selon les étapes suivantes :
- isolement des ARN de lymphocytes périphériques d'un individu ;
- obtention de l'ADN complémentaire à l'aide de reverse transcriptase et d'une amorce A spécifique de la région Cα (SEQ ID N° 21) ;
- amplification génique (par Anchored Polymerase Chain Reaction ou A-PCR) à l'aide d'une ADN polymérase, d'une amorce poly C (SEQ ID N° 22) et d'une amorce B spécifique de la région Cα (SEQ ID N° 23) ;
- nouvelle amplification par A-PCR à l'aide de ADN polymérase et d'une amorce C spécifique de la région Cα (SEQ ID N° 24) ;
- insertion dans un vecteur plasmidique ;
- transformation d'un hôte bactérien avec le vecteur recombinant ;
- criblage des colonies bactériennes recombinantes avec un oligonucléotide D spécifique de Cα (SEQ ID N° 25) marqué ;
- extraction des plasmides des colonies positives,
- et séquençage des fragments d'ADN contenant la région Cα.

La présente invention peut être reproduite notamment par amplification génique bispécifique (polymerase chain reaction ou PCR) en partant des lymphocytes périphériques expriment les ARNm incluant le segment variable correspondant au séquence ID N° 1 de l'invention ou alternativement en appliquant cette technique de PCR à de l'ADN génomique de toute cellule somatique d'un individu pris au hasard. L'invention peut aussi bien être reproduite en préparant le séquences géniques ci-dessus par synthèse chimique d'oligonucléotides.

Les peptides selon l'invention peuvent être obtenus par synthèse peptidique classique. Ils peuvent être également obtenus par application des techniques connues de génie génétique comprenant l'insertion d'une séquence d'ADN codant pour un peptide selon l'invention dans un vecteur d'expression tel qu'un plasmide et la transformation de cellules avec ce vecteur d'expression.

La présente invention a donc également pour objet des plasmides et des vecteurs d'expression comprenant une séquence d'ADN codant pour un peptide selon l'invention ainsi que des hotes transformés avec ce vecteur.

La présente invention a également pour objet des anticorps, et notamment des anticorps monoclonaux, dirigés contre un déterminant antigénique appartenant à ou comprenant un peptide selon l'invention.

Les anticorps monoclonaux peuvent être obtenus par toutes les techniques qui permettent la production de molécules d'anticorps à partir de culture de lignée cellulaire. Ces techniques comprennent les différentes techniques utilisant des hybridomes.

La production d'anticorps peut être obtenue chez l'animal par immunisation des animaux par injection des peptides ou des fragments selon l'invention, qu'ils soient naturels, recombinants ou synthétiques, éventuellement après couplage à un agent immunogène tel que l'anatoxine tétanique, ou encore par injection des lymphocytes T humains exprimant les séquences correspondantes à leur surface, y compris des cellules recombinantes transfectées avec les séquences codantes correspondantes.

La présente invention a également pour objet des hybridomes produisant des anticorps monoclonaux dirigés contre les polypeptides selon l'invention.

La présente invention englobe également les fragments et les dérivés d'anticorps monoclonaux selon l'invention qui sont réactifs avec des régions variables définies des récepteurs des cellules T. Ces fragments sont notamment les fragments F(ab')2 qui peuvent être obtenus par clivage enzymatique des molécules d'anticorps avec la pepsine, les fragments Fab' qui peuvent être obtenus par réduction des ponts disulfure des fragments F(ab')2 et les fragments Fab qui peuvent être obtenus par clivage enzymatique des molécules d'anticorps avec la papaïne en présence d'un agent réducteur. Ces fragments peuvent être également obtenus par génie génétique.

Les dérivés d'anticorps monoclonaux sont par exemple des anticorps ou des fragments de ces anticorps auxquels sont liés des marqueurs tel qu'un radioisotope. Les dérivés d'anticorps monoclonaux sont également des anticorps ou des fragments de ces anticorps auxquels sont liées des molécules thérapeutiquement actives, notamment des composés cytotoxiques.

Les produits de l'invention trouvent plusieurs types d'application dans le domaine du diagnostic et dans le domaine de la thérapeutique.

### 1 - Les applications dans le domaine du diagnostic

Les oligonucléotides contenus dans les séquences nucléotidiques selon l'invention peuvent être utilisés pour constituer des sondes de détection (généralement au moins 10 nucléotides) capables de s'hybrider à une région variable de chaîne α ou des amorces pour l'amplification d'ADN (comportant généralement au moins 15 nucléotides et de préférence au moins 17 nucléotides) capables de se lier à une séquence à amplifier.

Les oligonucléotides trouvent ainsi une application dans le diagnostic des désordres immunitaires en détectant la présence de séquences d'acides nucléiques homologues d'un gène codant pour des régions variables de chaînes α des récepteurs des cellules T dans l'ARNm d'un échantillon d'un patient. Différentes méthodes peuvent être utilisées pour établir un lien entre l'expression des gènes des cellules T et une maladie. Ces méthodes comprennent :
a - la production et l'analyse de banques d'expressions d'ADNc obtenu à partir de cellules T liées à la maladie pour déterminer la fréquence de gènes dominants ;
b - l'analyse d'échantillons d'ADN génomique par Southern blot pour déterminer s'il existe des polymorphismes génétiques ou des réarrangements des gènes codant pour les récepteurs des cellules T ;
c - l'analyse d'échantillons par obtention d'ADNc, amplification par PCR et hybridation avec des sondes marquées ;
d - l'hybridation in situ de cellules T sans culture préalable des cellules T.

Les amorces trouvent une application dans des réactions de PCR dans une méthode telle que celle définie sous c ci-dessus.

Les anticorps monoclonaux, les fragments ou les dérivés de ces anticorps selon l'invention, notamment les anticorps anti Vα, peuvent être utilisés pour étudier des réponses immunitaires de type T, par exemple dans le domaine des maladies auto-immunes de la cancérologie, de l'allergie, de la transplantation et des maladies infectieuses. En particulier, le répertoire des différents segments variables a du récepteur T peut être étudié, qu'il s'agisse de cellules T du sang ou des tissus. D'une manière générale, les techniques utilisées peuvent être des méthodes in vitro ou in vivo.

Dans les méthodes in vitro, les échantillons utilisés peuvent être des échantillons de fluides corporels ou des échantillons de tissus. Les techniques utilisées peuvent inclure notamment la cytofluorimétrie de flux pour analyser les lymphocytes T du sang ou des marquages par immunopéroxydase sur coupe anatomopathologique pour étudier les lymphocytes infiltrant les tissus.

Dans les méthodes in vivo, les anticorps, leurs fragments ou leurs dérivés sont administrés par les voies habituelles, par exemple par la voie intraveineuse, et l'on détecte les liaisons immunospécifiques. Ceci peut être obtenu par exemple dans le cas où l'on utilise un anticorps marqué par un radioisotope.

### 2 - Les applications dans le domaine thérapeutique

Les oligonucléotides contenus dans les séquences nucléotidiques selon l'invention peuvent être utilisés en thérapeutique comme oligonucléotides antisens. On sait en effet qu'il est possible in vitro d'inhiber l'expression d'un gène transcrit dans des lymphocytes humains en incubant ces lymphocytes avec un oligonucléotide antisens spécifique du gène en question (26). Ces oligonucléotides antisens comprennent généralement au moins 10 et, de préférence, au moins 16 nucléotides. Ces oligonucléotides antisens peuvent être notamment les séquences inversées et complémentées correspondant aux 20 nucléotides en amont du site d'initiation de la traduction (ATG). L'intérêt d'une utilisation in vitro d'oligonucléotides antisens spécifique d'un segment génique Vα est d'abolir (ou de diminuer fortement) l'expression d'un récepteur T comprenant ce segment Vα et donc d'obtenir un phénomène de délétion clonale au niveau de la réactivité spécifique des lymphocytes T. Les oligonucléotides antisens peuvent non seulement être utilisés in vitro sur des lymphocytes T humains qui sont ensuite réinjectés, mais également in vivo par injection locale ou systémique de préférence après modifications pour augmenter la stabilité in vivo et la pénétration à l'intérieur des lymphocytes T de ces oligonucléotides.

Les anticorps monoclonaux selon l'invention, notamment les anticorps anti Vα, peuvent être utilisés pour moduler le système immunitaire. C'est ainsi que les anticorps peuvent être administrés pour bloquer l'interaction des cellules T effectrices avec leur antigène spécifique. On peut également administrer des anticorps anti-récepteurs T liés par exemple à une molécule cytotoxique ou à un radioisotope de façon à obtenir une délétion clonale, grâce à la fixation spécifique sur une chaîne α d'un récepteur de cellule T. Les anticorps monoclonaux selon l'invention peuvent être utilisés en thérapeutique à des concentrations faiblement mitogéniques de façon à activer de façon spécifique certains sous-ensembles de cellules T ou peuvent être utilisés à des concentrations beaucoup plus élevées pour se fixer aux récepteurs concernés et ainsi marquer ces sous-ensembles en vue de leur élimination par le système réticulo-endothélial. Un critère important pour le traitement d'une maladie est l'aptitude à moduler des sous-ensembles de cellules T liées à une maladie. La nature exacte de cette modulation thérapeutique, à savoir bloquer ou supprimer un sous-ensemble particulier de cellules T ou au contraire stimuler et activer un sous-ensemble particulier, dépendra de la maladie en question et du sous-ensemble spécifique de cellules T concerné.

Ce type de traitement présente un avantage par rapport aux traitements actuels utilisant des anticorps tels que le traitement par des anticorps anti CD3 chez des patients ayant subi une transplantation rénale et ayant un problème de rejet, étant donné que grâce à l'invention il n'y aura pas de modulation de la totalité de la population des cellules T mais seulement du sous-ensemble de cellules T exprimant la sous-famille α particulière de récepteurs de cellules T.

Par ailleurs, la réponse des cellules T étant souvent oligoclonale, il convient généralement d'utiliser en thérapeutique des "cocktails" de plusieurs anticorps.

On peut en outre utiliser les anticorps anti Vα pour sélectionner in vitro des lymphocytes T, par exemple par passage sur une colonne contenant des billes portant l'anticorps. Cette séparation de certains lymphocytes T peut être utilisée en vue d'une mise en culture de ces lymphocytes avant de les réinjecter au patient.

En outre, on peut utiliser en thérapeutique tout ou partie des séquences peptidiques selon l'invention, c'est-à-dire les séquences peptidiques codées par les séquences nucléotidiques selon l'invention ou les fragments de ces séquences (comprenant généralement au moins 8 à 10 amino-acides). Ces séquences ou ces fragments, administrés à l'homme ou à l'animal, peuvent agir en tant que leurre, c'est-à-dire qu'ils vont se fixer sur l'épitope porté par l'antigène néfaste et empêcher la réaction des cellules T normales avec l'antigène, en empêchant ainsi le développement d'une maladie agressive contre les déterminants du soi. Ils peuvent aussi être utilisés en tant qu'immunogènes dans la fabrication de vaccins (éventuellement après couplage à des porteurs protéiques).

On décrira ci-après plus en détail la présente invention, en se reportant aux Fig. annexées sur lesquelles :

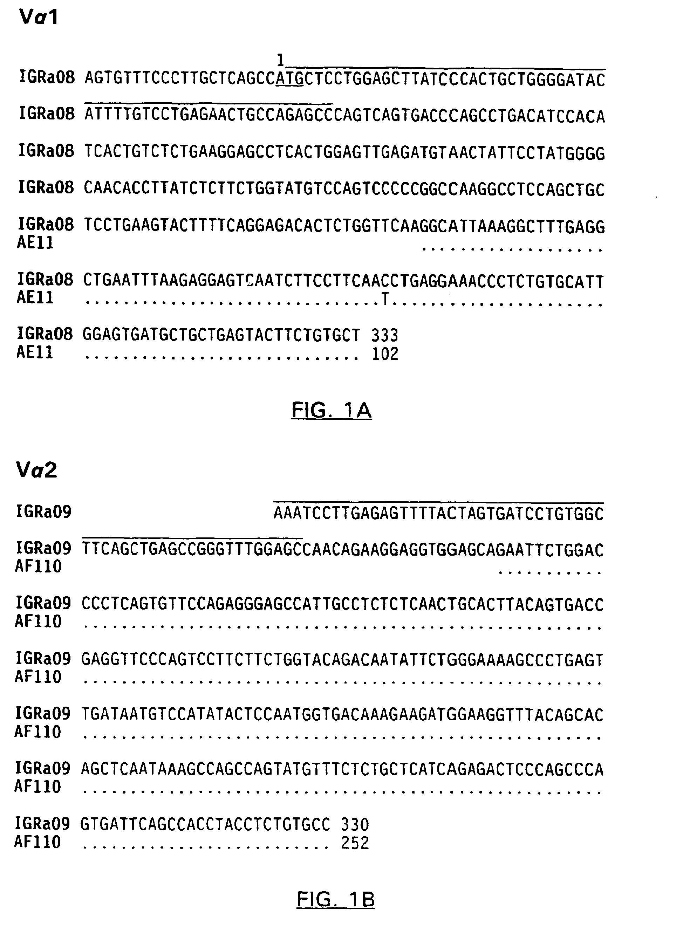

- les Fig. 1 A à E donnent en alignement à la fois une séquence Vα connue et une séquence partielle d'une extension selon l'invention pour les séquences respectives SEQ ID N° 6 à 10, notées IGRa 08 à IGRa 12. Sur ces Figures, la numérotation des nucléotides commence au codon ATG d'initiation (qui est souligné). Les points indiquent les nucléotides identiques. Les séquences qui sont supposées être des séquences leader sont surlignées.

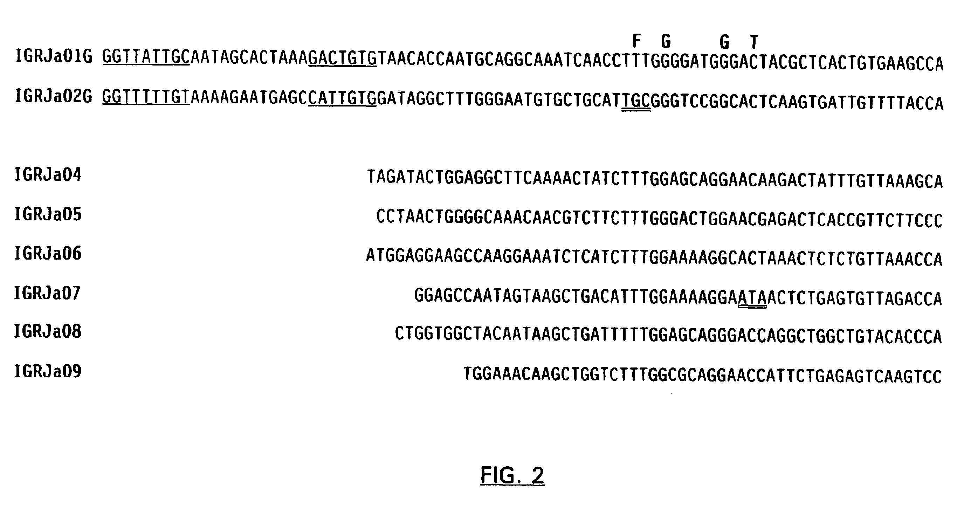

- La Fig. 2 donne en alignement les nouvelles séquences Jα (SEQ ID N° 12, 13 et 15 à 20) notées IGRJa 01, 02 et 04 à 09. Dans ces séquences les signaux de recombinaison de la lignée germinale sont soulignés. Les aminoacides correspondant aux codons hautement conservés sont marqués au-dessus des séquences. Les codons correspondant à une substitution en une position d'un aminoacide conservé sont doublement soulignés.

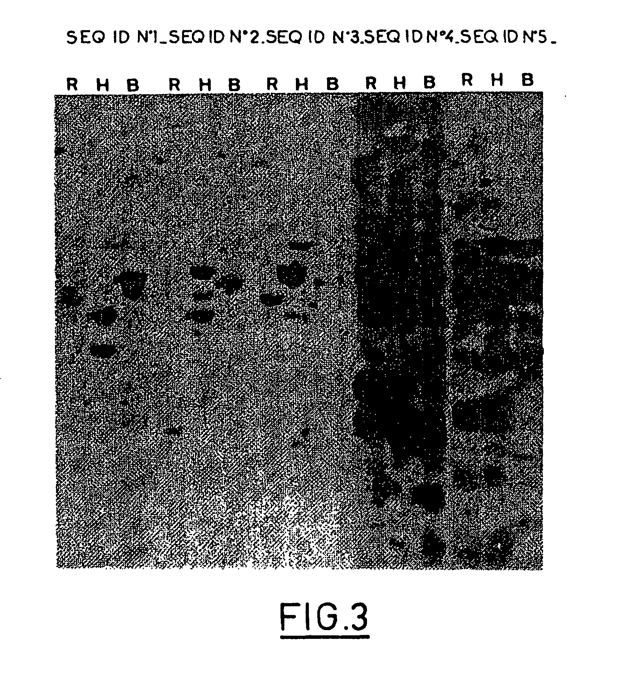

- La Fig. 3 donne les analyses par Southern blot de l'ADN génomique traité par une enzyme de restriction en utilisant des sondes spécifiques des séquences SEQ ID N° 1 à 5. Les enzymes de restriction utilisées sont EcoRI (colonne R), Hind III (colonne H) et Bam III (colonne B). Sur cette Figure, les triangles marquent la position des fragments d'ADN s'hybridant de façon spécifique avec Cα.

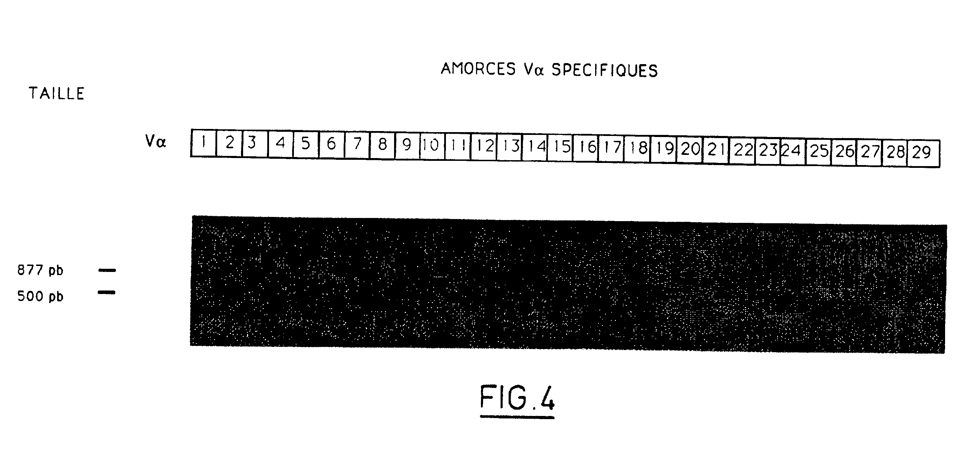

- La Fig. 4 représente la détection par autoradiographie des transcrits amplifiés des chaînes α du TCR exprimé par les lymphocytes périphériques d'un individu sain et du contrôle β-actine, co-amplifié.

### I - Obtention de l'ADNc et amplification par PCR

On a utilisé comme source d'ARN des lymphocytes périphériques d'un individu. L'ARN total a été préparé selon la méthode à l'isothiocyanate de guanidinium et au chlorure de césium (Chirgwin (10)) ou selon la méthode en une seule étape par extraction avec de l'isothiocyanate de guanidinium, du phénol et du chloroforme (Chomczynski (11)).

Le premier brin d'ADNc a été synthétisé dans un volume final de 50 microlitres à une température de 42°C pendant 1 heure en utilisant 5 microgrammes d'ARN total, la reverse transcriptase et une amorce A spécifique de la région Cα constituée par la séquence 5'-GTTGCTCCAGGCCACAGCACTG (SEQ ID N° 21). Ce matériau a été ensuite purifié par extraction au phénol/chloroforme et précipitation à l'acétate d'ammonium. Après sélection d'une fraction 0,45/1 kb sur gel d'agarose, on a réalisé l'addition d'une extrémité dG sur l'hétéro duplex ARN/ADNc dans un tampon d'addition CoCl2 avec 14 unités de terminal désoxynucléotidyl transférase (Tdt) pendant 30 mn à 37° C. La réaction a été stoppée par maintien à 70° C pendant 10 mn. NaOH 1N (1/3 de volume) a été ajouté et l'échantillon a été mis à incuber à 50° C pendant 1 heure pour hydrolyser l'ARN puis a été neutralisé avec du Tris HCl 2M pH 8 et HCl 1N. Après extraction par un mélange phénol/ chloroforme le premier brin d'ADNc à extrémité G a été précipité avec de l'éthanol et soumis à une amplification en utilisant la technique PCR (Polymerase Chain Reaction décrite par Saiki et al. (12)) dans un volume final de 100 microlitres contenant 50 mM de KCl, 10 mM de Tris-Cl pH 8.3, 1,5 mM de MgCl2, 0,1 % (poids/ volume) de gélatine, 200 micromoles de dNTP, 2,5 unités de Taq polymérase et 100 picomoles de deux amorces. Les deux amorces utilisées sont, d'une part, une amorce poly-C (5'-GCATGCGCGCGG CCGCGGAGG-14C) (SEQ ID N° 22) décrite par Loh et al. (13) ainsi qu'une amorce B spécifique de la région Cα (5'-GTCCATAGACCTC ATGTCCAGCACAG) (SEQ ID N° 23).

On effectue 25 cycles d'amplification suivis par une période de 15 mn d'élongation finale à 72° C. Chaque cycle comprend une étape de dénaturation à 92° C pendant 1 minute, une étape d'hybridation à 55° C pendant 2 mn et une période d'élongation à 72° C pendant 4 mn. Les produits amplifiés sont ensuite précipités par de l'éthanol, remis en suspension dans de l'acétate de sodium 30 mM pH5, NaCl 50 mM, ZnC12 1 mM, glycérol 5 % en volume, et 1/10 de ce matériau est purifié en fonction de la taille sur un gel d'agarose à bas point de fusion 1 %.

Une seconde phase d'amplification est ensuite réalisée directement sur approximativement 10 % de la bande contenant l'agarose en suivant les mêmes conditions que précédemment, sauf qu'on utilise comme amorce C spécifique de la région Cα l'amorce 5'-ATACACATCAGAATTC TTACTTTG (SEQ ID N° 24). Le mélange de réaction est ensuite précipité par l'éthanol et remis en suspension dans 60 µl H2O.

### II - Clonage et séquençage des ADNc

1/3 du produit de la seconde amplification est mis à digérer avec Sac II, séparé sur gel d'agarose 1 % et purifié par adsorption sur des billes de verre. Le matériau est inséré dans le vecteur Bluescript SK+ (Stratagene, La Jolla, U.S.A.) et les recombinants obtenus sont utilisés pour transformer des souches XL1-bleu de E. Coli (Stratagene). Après dépôt en présence de X-gal et IPTG, on fait un test sur les colonies blanches en utilisant une technique "dot blot" et comme sonde un troisième oligonucléotide spécifique de la région Cα (5'-GTCACTGG ATTTAGAGTCT) (SEQ ID N° 25) marqué au ³²P. On extrait l'ADN plasmidique des colonies positives et on séquence sur les deux brins par le procédé par terminaison de chaîne didésoxy (Sanger et al. (14)) avec de la Sequenase 2,0 (United States Biochemicals, Cleveland, Etats-Unis) en suivant les recommandations du fournisseur. Sauf pour la séquence SEQ ID N° 5, toutes les séquences nucléotidiques ont été déterminées sur les deux brins en utilisant au moins deux clones distincts d'ADNc.

Les séquences obtenues ont été comparées aux séquences publiées Vα et Jα en utilisant la méthode développée par Lipman et Pearson (15). Les codons de départ présumés ont été identifiés en recherchant la présence de la séquence consensus Kozak pour les sites d'initiation des traductions dans les cellules eucaryotes (Kozak (16)). La présence de séquences leader hydrophobes du côté N-terminal a été décelée par analyse de l'hydrophobicité selon la méthode décrite par Kyte (17).

### III - Analyse par Southern blot

L'ADN a été extrait de la lignée cellulaire érythroleucémique humaine K562 et mis à digérer avec l'une des enzymes de restriction suivantes : EcoR I, BamH I ou Hind III. L'ADN (15 microgrammes) a été soumis à une électrophorèse sur agarose 0,7 % et a été transféré sur des membranes de Nylon comme décrit par Triebel et al. (18). Les hybridations ont été réalisées à 65° C avec 6 x SSC, 0,5 % de SDS, 5 x Denhardt's et 100 microgrammes d'ADN de sperme de saumon dénaturé pendant 16 heures. Les membranes ont été lavées à 65° C avec 2 x SSC, 0,2 % de SDS.

On a utilisé comme sondes Vα spécifiques des sondes obtenues par amplification d'ADNc V-J-C (> 500 bp) comprenant des fragments Vα correspondant aux séquences SEQ ID N° 1 à 5 en utilisant comme amorce l'amorce poly-C et l'amorce C. Les sondes ont été purifiées sur gel d'agarose 1 %. Des sondes d'ADN marquées au ^{**32**}P ont été préparées à partir des fragments purifiés sur agarose par la méthode de Feinberg (19).

### IV - Résultats

En utilisant la méthode A-PCR, 308 ADNc qui hybrident avec la sonde Cα ont été clones, puis séquences. Parmi ceux-ci, 172 ADNc correspondent à des régions variables V-J-Cα uniques.

Les séquences Vα et Jα décrites figurent dans la liste des séquences respectivement sous les SEQ ID N°1 à 11 et SEQ ID N° 12, 13 et 15 à 20. Les séquences SEQ ID N° 2 à 5 correspondent à des sous familles nouvelles (dénommées respectivement Vα 25, Vα 26, Vα 27 et Vα 29) tandis que les séquences SEQ ID N° 1 et 6 à 11 correspondent à des extensions de segments Vα connus.

### 1. Séquences Vα correspondant à des sous-familles nouvelles

Les analyses par Southern blot d'ADN de lignée germinale soumis à une digestion par les endonucléases, en utilisant des sondes V-J-Cα comprenant des fragments Vα correspondant aux séquences SEQ ID N° 2 à 5 ont été réalisées dans des conditions d'hybridation de "faible stringence" pour identifier le nombre de segments géniques Vα appartenant à chaque famille et pour caractériser des fragments de restriction d'ADN portant ces segments géniques Vα. Des résultats représentatifs sont reportés sur la Figure 3.

Ces analyses montrent que la sous famille correspondant à la séquence SEQ ID N° 3 comprend au moins deux segments géniques alors que les autres séquences (SEQ ID N° 2, N° 4 et N° 5) correspondent probablement à des membres uniques.

Les tailles des fragments de restriction de l'ADN germinal sont les suivantes :
SEQ ID N° 2 : EcoR I 2,2 kb, Hind III 4,8 et 5,7 kb, BamH I 25 kb
SEQ ID N° 3 : EcoR I 4,6 et 7,5 kb, Hind III 4,2 et 6,4 kb, BamH I 23 et 4,5 kb
SEQ ID N° 4 : EcoR I 7,6 kb, Hind III 18 kb, BamH I 9 et 0,9 kb
SEQ ID N° 5 : ECOR I 5,9 et 4,8 kb, Hind III 6,6 kb, BamH I 6,5 kb.

### 2. Séquences correspondant à des extensions de séquences Vα connues

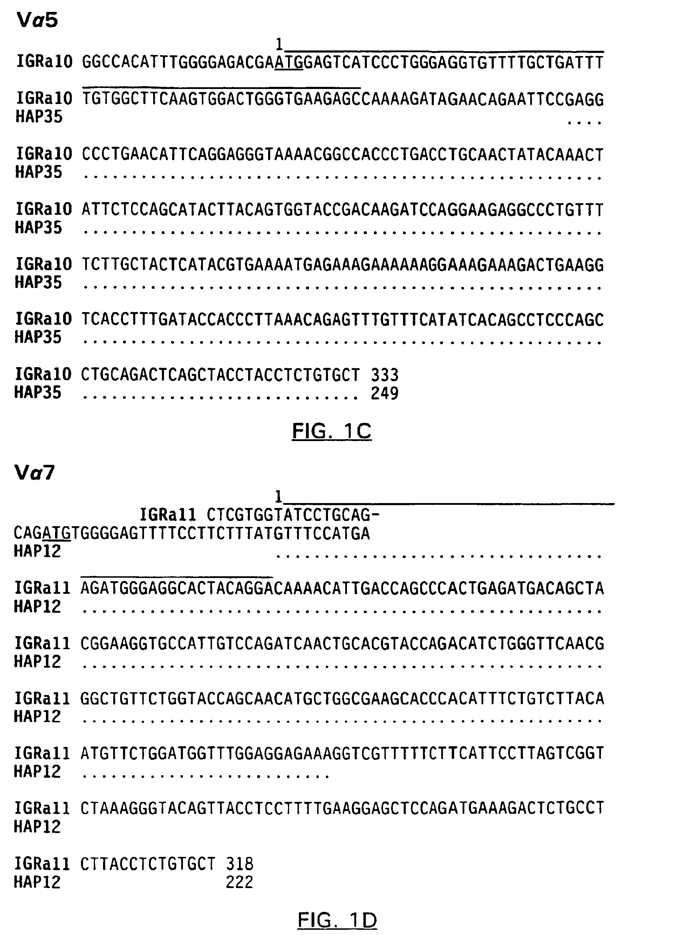

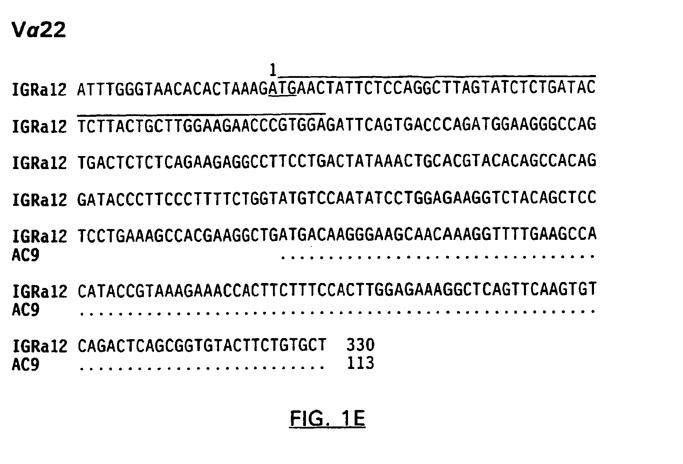

SEQ ID N° 1 (IGR a 02) correspond à une extension du côté 5' de la séquence LINV (171 bp) (Mengle-Gaw (20)) : Cette séquence définit la sous-famille dénommée provisoirement Vα w24.
SEQ ID N° 6 (IGR a 08) : Cette séquence correspond à une extension du côté 5' de la séquence du clone Va1 AE11 (Klein et al. (21)). Les deux séquences alignées sont représentées sur la Figure 1A.
SEQ ID N° 7 (IGR a 09) : Cette séquence correspond à une extension codant pour l'extrémité NH2 terminale de la séquence Vα2 AF110 (Klein déjà cité). Les deux séquences alignées sont représentées sur la Fig. 1B. La séquence ID N° 7 correspond à une séquence consensus. On a observé en position 206 l'existence d'un T au lieu d'un C.
SEQ ID N° 8 (IGR a 010) : Cette séquence correspond à une extension de la région 5' du clone Vα HAP35 (Yoshikai (22)). Les deux séquences alignées sont représentées sur la Fig. 1C. La séquence ID N° 8 correspond à une séquence consensus. On a observé en position 307 l'existence d'un G au lieu d'un A et en position 360 l'existence d'un T au lieu d'un C.
SEQ ID N° 9 (IGR a 11) : Cette séquence correspond à une extension du côté 3' de la séquence Vα7 HAP12 (Yoshikai déjà cité). L'alignement des séquences est représenté sur la Fig. 1D. La séquence ID N° 9 correspond à une séquence consensus. On a observé en position 86 l'existence d'un C au lieu d'un T. SEQ ID N° 10 (IGR a 12) : Cette séquence comprend la région codante complète d'un gène de la sous famille Vα 22 qui précédemment avait été identifié par la séquence partielle (113 bp) AC9 (Klein déjà cité). Les deux séquences alignées sont représentées sur la Fig. 1E.
SEQ ID N° 11 (IGRa 13) : Cette séquence correspond pour une partie aux clones HAVT 32 et HAVT 35 (appartenant à la sous-famille Vα 16 (8) et qui ont été décrits comme des pseudogènes. En fait, par suite de l'addition d'un nucléotide en position 108, la SEQ ID N° 11 code pour une région variable originale d'un récepteur des lymphocytes T. Par ailleurs, cette séquence est homologue d'une séquence HSTCAYM (Klein et al. (21)) pour la partie codante. Toutefois la séquence SEQ ID N° 11 est la seule qui soit complète et codante.

### 3. Séquences Jα

On a représenté sur la Fig. 2 l'ensemble des nouvelles séquences Jα. Parmi les 8 segments Jα, la plupart d'entre eux présentent une séquence d'aminoacides hautement conservée FGXGT des segments Jα comme décrit par Yoshikai déjà cité. Cependant, pour le segment IGRJa 07 le résidu thréonine est remplacé par un résidu isoleucine.

De plus, à la place du résidu phénylalanine, on a trouvé un résidu cystéine dans IGRJa 02G.

L'expression prédominante de certaines sous-familles de Vα a déjà été étudiée à l'aide d'une gamme incomplète d'oligonucléotides. Ainsi, Nitta et al. (29) ont décrit l'expression prédominante des gènes Vα 7 dans les lymphocytes infiltrant les tumeurs. Par ailleurs, Sottini et al. (30) ont décrit chez des patients atteints d'arthrite rhumatoïde l'étude du répertoire des Vα.

Une gamme complète d'oligonucléotides permettant l'étude à la fois des sous-familles Vα connues et des nouvelles sous- familles Vα et qui soient tout-à-fait spécifiques de chaque sous-famille est décrite. Des oligonucléotides ont donc été choisis et synthétisés dans ce but et au besoin des modifications de un ou deux nucléotides ont été introduites par rapport aux séquences naturelles pour réduire les réactivités croisées entre sous-familles.

La présente invention a ainsi également pour objet un oligonucléotide, utilisable comme amorce pour l'amplification d'ADN correspondant à une région variable de chaîne α des récepteurs de cellules T, (de sequence SEQ ID N° 49.

La présente invention a également pour objet l'utilisation, comme amorce pour l'amplification d'ADN correspondant à Une région variable de chaîne a des récepteurs de cellules T, d'un oligonucléotide de sequence SEQ ID N° 49.

La présente invention a également pour objet un procédé de détection de séquences nucléotidiques codant pour des segments Vα des récepteurs T ou d'ADNc correspondant aux produits de transcription de celles-ci, dans un échantillon biologique, caractérisé en ce qu'il comprend :
a) l'amplification de l'ADN avec au moins une paire d'amorces formée par un oligonucléotide de séquence SEQ ID N° 49 et et un oligonucléotide appartenant au segment Cα, et
b) la détection des séquences amplifiées avec une sonde Cα.

L'oligonucléotide appartenant au segment Cα utilisé pour l'amplification peut être notamment choisi parmi les séquences SEQ ID N° 55 et 56.

Pour contrôler l'efficacité de l'amplification, on opère avantageusement en présence d'une paire d'amorces contrôle et on détecte la séquence contrôle correspondante amplifiée à l'aide d'une sonde contrôle correspondante.

Cette paire d'amorces contrôle peut correspondre à deux segments Cβ, par exemple les amorces CβF et CβK correspondant aux séquences SEQ ID N° 61 et 62. On utilise alors une sonde de détection Cβ (correspondant par exemple à la séquence SEQ ID N° 63). Mais cette paire d'amorces peut être également constituée par deux amorces appartenant à la β-actine, notamment celles correspondant aux séquences SEQ ID N° 58 et 59. On utilise alors une sonde de détection correspondant à une séquence de la β-actine, telle que la séquence SEQ ID N° 60.

La présente invention a également pour objet un kit de diagnostic pour la mise en oeuvre du procédé précédemment défini, qui comprend :
a) au moins un oligonucléotide de séquence SEQ ID N° 49,
b) une amorce Cα,
c) une sonde Cα.

Un tel kit contient avantageusement en outre:
d) une paire d'amorces contrôle,
e) une sonde contrôle.

Dans l'information donnée dans la liste des séquences pour les séquences 26 à 60, les séquences SEQ ID N° 26 à 47 correspondent à des séquences appartenant à des clones de sous-familles connues Va1 à Vα 22 (accessibles dans la base de données EMBL) ou à des séquences qui en diffèrent par un ou deux nucléotides.

Les séquences SEQ ID N°49, 50, 51, 52 et 54 correspondant à des séquences appartenant à des clones de nouvelles sous-familles de l'invention, correspondent à des sous-familles dénommées provisoirement Vα · w24, Vα w25, Vα w26, Vα w27 et Vα w29 (w indiquant que la désignation est dans l'attente d'une désignation définitive).

Les séquences SEQ ID N° 48 et 53 correspondent à des séquences appartenant à des clones respectivement IGRa01 et IGRa06 de sous-familles connues mais n'ayant pas encore reçu de désignation définitive (respectivement Vα w23 et Vα w28) dont un élément membre a déjà été décrit (respectivement Hinkkanen A. et al. (31) et Bernard O. et al. (32)). La séquence complète de IGRa06 n'a pas encore été publiée.

Les séquences SEQ ID N° 55 et 56 sont deux exemples d'oligonucléotides qui peuvent être utilisés comme amorces Cα pour l'amplification.

La séquence SEQ ID N° 57 est la séquence d'une sonde Cα qui peut être utilisée pour la détection des ADN amplifiés.

Les séquences SEQ ID N° 58, 59 et 60 sont respectivement les séquences d'une paire d'oligonucléotides appartenant à la séquence de la β-actine qui peuvent être utilisées pour le contrôle de l'amplification et la séquence d'une sonde pour détecter les ADN amplifiés correspondants.

Dans la liste des séquences, la position indiquée est la position de l'extrémité 5' à compter du site d'initiation prédictif de la traduction ATG. Dans le cas où les séquences sont incomplètes (région 5' inconnue), la position (marquée avec un astérisque) est donnée par rapport au premier nucléotide de la séquence. Les nucléotides soulignés correspondent aux mésappa-riements introduits par rapport à la séquence naturelle.

Les oligonucléotides ont été synthétisés sur un synthétiseur d'ADN automatisé Applied Biosystems 381 A en utilisant la méthode au *β*-cyanoéthylphosphoramidite (Sinha et al. (33)) et en suivant le protocole recommandé par le fabricant. Les oligonucléotides ont été détritylés dans l'appareil, clivés du support et déprotégés par l'ammoniac (à 60° C pendant 5 heures). Les produits bruts ont été purifiés par chromatographie haute pression en phase inversée sur une colonne de *µ*-bondapak C 18 en utilisant un gradient d'acétonitrile (9 à 15 %) dans un tampon acétate de triéthylammonium 0,01 M à pH 5,5.

L'amplification effectuée en utilisant les amorces selon l'invention peut être notamment la technique par PCR (Polymerase Chain Reaction) telle que décrite par Saiki et al. (12) et brevets US-A-4 683 195, 4 683 202, 4 889 818.

Pour la PCR, on peut utiliser un ADN double brin que l'on dénature ou un ADNc obtenu à partir d'ARN à l'aide de réverse transcriptase comme mentionné plus haut.

L'agent de polymérisation est une ADN polymérase, notamment la Taq polymérase.

Généralement, le cycle d'amplification est répété 25 à 40 fois.

Les sondes que l'on utilise pour la détection des séquences amplifiées peuvent être obtenues par marquage d'oligonucléotides avec un isotope radioactif, ce qui conduit à une détection par autoradiographie, ou par couplage avec une enzyme telle que la peroxydase (système ECL Amersham), la phosphatase alcaline ou la β-galactosidase (système Tropix Ozyme), ce qui conduit à une détection par chimioluminescence.

L'exemple suivant illustre la mise en oeuvre du procédé de détection selon l'invention.

Les lymphocytes périphériques d'un individu sain ont été préparés par centrifugation sur un gradient de densité. L'ARN total a été extrait selon la méthode en une seule étape par extraction avec de l'isothiocyanate de guanidium, du phénol et du chloroforme (Chomczynski, 11). L'ADN complémentaire a été synthétisé dans un volume final de 20 *µ*l à 42° C durant 1 heure en utilisant 1 à 5 *µ*g d'ARN total, la réverse transcriptase et l'amorce Cα B (1,25 *µ*M).

Le matériel obtenu a ensuite été chauffé à 95° C durant 3 minutes avant d'être soumis à une amplification selon la technique PCR en utilisant en parallèle chacune des amorces Vα spécifique correspondant aux séquences SEQ ID N° 26 à 54 et l'amorce Cα B spécifique de la région Cα (SEQ ID N° 56). Cette amplification a été réalisée dans un volume final de 10 *µ*l par tube contenant 50 mM de KCl, 10 mM de tris-HCl pH 8,3, 1,5 mM de MgCl2 0,1 % (poids/ volume) de gélatine, 200 *µ*M de dNTP, 0,25 unités de Taq polymérase et 0,25 *µ*M de chaque amorce. Dans chaque tube, une amplification contrôle a été réalisée à partir de 25 mM d'un fragment d'ADN de *β*-actine de 877 paires de bases préparé par PCR et des amorces Act 1 et Act 2 (SEQ ID N° 58 et 59) spécifiques de l'actine. 30 cycles d'amplification ont été effectués suivis d'une étape d'élongation finale de 5 minutes à 72° C. Chaque cycle comprenait une étape de dénaturation à 94° C pendant 1 minute, une étape d'hybridation à 65° C pendant 1 minute et une période d'élongation à 72° C pendant 1 minute.

Les produits obtenus ont été séparés par électrophorèse sur un gel d'agarose à 2 %, transférés sur des membranes de nylon dans un tampon alcalin et hybridés simultanément avec les sondes oligonucléotides Cα C (SEQ ID N° 57) et Act 3 (SEQ ID N° 60) marquées au 32P par l'enzyme polynucléotidyl T4 kinase. L'hybridation a été réalisée à 42° C pendant 16 heures dans un tampon contenant 6 x SSC, 0,5 % SDS, 5 x Denhart's, 0,05 % P04H2Na et 100 *µ*g/ml d'ADN de sperme de saumon dénaturé. Les membranes ont ensuite été lavées avec du 6 x SSC, 20mM P04H2Na, deux fois à température ambiante pendant 5 minutes et une fois à 50° C pendant 30 minutes puis autoradiographiées.

Les résultats sont montrés sur la figure 4.

Le contrôle d'actine (bande de 877 paires de bases) permet de vérifier l'amplification dans tous les puits. Il apparaît en-dessous de cette bande un signal spécifique dont la taille correspond à la taille des fragments amplifiés correspondants, chaque fragment ayant une longueur correspondant à la distance entre l'emplacement de l'oligonucléotide spécifique Vα et l'amorce Cα.

Chez l'individu testé, la Figure 4 met en évidence l'expression préférentielle de certains segments géniques définis par rapport à d'autres. Par exemple, les sous-familles Vα 27, 28 et 29 sont moins bien représentées que les sous-familles Vα 2, 3 et 6.

### REFERENCES :

1. Meuer, S.C., et al., J. Exp. Med. 1983. 157:705.
2. Moingeon, P., et al., Nature 1986a. 323:638.
3. Brenner, M.B., et al., Nature 1986. 322:145.
4. Bank, I., et al., Nature 1986. 322:179.
5. Davis, M.M., et al., Nature 1988.334:395.
6. Crews, S., et al., Cell 1981. 25:59.
7. Concannon, P., et al., Proc. Natl. Acad. Sci. USA 1986. 83:6598.
8. Kimura, N., et al., Eur. J. Immunol. 1987. 17:375.
9. Wilson, R.K., et al., Immunological Reviews 1988c. 101:149.
10. Chirgwin, J.M., et al., Biochemistry 1979. 18:5294.
11. Chomczynski, P. et al., Anal. Biochem. 1987, 162:156.
12. Saiki, R.K., et al., Science 1988. 239:487.
13. Loh, E.Y., et al., Science 1989. 243:217.
14. Sanger, F., et al., Proc. Natl. Acad. Sci. USA 1977. 74:5463.
15. Lipman, D.J., et al., Science 1985. 227:1435.
16. Kozak, M., Nucl. Acids Res. 1984. 12:857.
17. Kyte, J., et al., R.F., J. Mol. Biol. 1982. 157:105.
18. Triebel, F., et al., J. Immun. 1988. 140:300.
19. Feinberg, A.P., et al., Anal. Biochem. 1983. 132:6.
20. Mengle-Gaw, L., et al., The EMBO Journal, 1987. 6:2273.
21. Klein, M.H., et al., Proc. Natl. Acad. Sci. USA 1987. 84:6884.
22. Yoshikai, Y., et al., J. Exp. med. 1986. 164:90.
23. Naudenbark A., et al., Nature, 341, 541.
24. Janeway C., Nature, 341, 482.
25. Lin Y., J. Exp. Med., 171, 221.
26. Acha-Orbea H., EMBO Journal, 1990,9, 12, 3815.
27. Kappler J., Science, 244, 811
28. Choi, Y., PNAS, 86, 8941.
29. Nitta T. et al., Science 1990, 249, 672.
30. Sottini A. et al., Eur. J. Immunol., 1991, 21, 461.
31. Hinkkanen A. et al., Immunogenetics, 1989, 29, 131.
32. Bernard O. et al., Oncogene, 1988, 2, 195.
33. Sinha N. et al., Nucleic Acids Res. 1984, 12, 4539.

### LISTE DES SEQUENCES

I - INFORMATION GENERALE
   (1) DEMANDEUR : Société dite ROUSSEL UCLAF
   (2) TITRE DE L'INVENTION :
      Séquences nucléotidiques codant pour des régions variables des chaînes α des récepteurs des lymphocytes T humains, segments peptidiques correspondants et les applications diagnostiques et thérapeutiques.
   (3) NOMBRE DE SEQUENCES : 62
II - INFORMATION POUR SEQ ID N° 1
   CARACTERISTIQUES DES SEQUENCES
      TYPE : nucléotide et sa protéine correspondante
      LONGUEUR : 371 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 02
      SEQUENCE Vα w24
   DESCRIPTION DE LA SEQUENCE 
AGTCAACTTC TGGGAGCAGT CTCTGCAGAA TAAAA ATG AAA AAG CAT 47
Met Lys Lys His
1

CTG ACG ACC TTC TTG GTG ATT TTG TGG CTT TAT TTT TAT AGG GGG AAT 95
Leu Thr Thr Phe Leu Val Ile Leu Trp Leu Tyr Phe Tyr Arg Gly Asn
5                   10                  15                  20

GGC AAA AAC CAA GTG GAG CAG AGT CCT CAG TCC CTG ATC ATC CTG GAG 143
Gly Lys Asn Gln Val Glu Gln Ser Pro Gln Ser Leu Ile Ile Leu Glu
                25                  30                  35

GGA AAG AAC TGC ACT CTT CAA TGC AAT TAT ACA GTG AGC CCC TTC AGC 191
Gly Lys Asn Cys Thr Leu Gln Cys Asn Tyr Thr Val Ser Pro Phe Ser
            40                  45                  50

AAC TTA AGG TGG TAT AAG CAA GAT ACT GGG AGA GGT CCT GTT TCC CTG 239
Asn Leu Arg Trp Tyr Lys Gln Asp Thr Gly Arg Gly Pro Val Ser Leu
        55                  60                  65

ACA ATC ATG ACT TTC AGT GAG AAC ACA AAG TCG AAC GGA AGA TAT ACA 287
Thr Ile Met Thr Phe Ser Glu Asn Thr Lys Ser Asn Gly Arg Tyr Thr
    70                  75                  80

GCA ACT CTG GAT GCA GAC ACA AAG CAA AGC TCT CTG CAC ATC ACA GCC 335
Ala Thr Leu Asp Ala Asp Thr Lys Gln Ser Ser Leu His Ile Thr Ala
85                  90                  95                  100

TCC CAG CTC AGC GAT TCA GCC TCC TAC ATC TGT GTG 371
Ser Gln Leu Ser Asp Ser Ala Ser Tyr Ile Cys Val
                105                 110
III - INFORMATION POUR SEQ ID N° 2
   CARACTERISTIQUES DES SEQUENCES
      TYPE: nucléotide et sa protéine correspondante
      LONGUEUR : 400 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 03
      SEQUENCE Vα w25
   DESCRIPTION DE LA SEQUENCE 
GACTCTAAGC CCAAGAGAGT TTCTTGAAGC AAAAAAAAAA 40

AAAACCCATT CAGGAAATAA TTCTTTGCTG ATAAGG ATG CTC CTT GAA CAT TTA 94
Met Leu Leu Glu His Leu
1               5

TTA ATA ATC TTG TGG ATG CAG CTG ACA TGG GTC AGT GGT CAA CAG CT[?] 142
Leu Ile Ile Leu Trp Met Gln Leu Thr Trp Val Ser Gly Gln Gln Leu
            10                  15                  20

AAT CAG AGT CCT CAA TCT ATG TTT ATC CAG GAA GGA GAA GAT GTC TCC 190
Asn Gln Ser Pro Gln Ser Met Phe Ile Gln Glu Gly Glu Asp Val Ser
        25                  30                  35

ATG AAC TGC ACT TCT TCA AGC ATA TTT AAC ACC TGG CTA TGG TAC AAG 238
Met Asn Cys Thr Ser Ser Ser Ile Phe Asn Thr Trp Leu Trp Tyr Lys
    40                  45                  50

CAG GAC CCT GGG GAA GGT CCT GTC CTC TTG ATA GCC TTA TAT AAG GCT 286
Gln Asp Pro Gly Glu Gly Pro Val Leu Leu Ile Ala Leu Tyr Lys Ala
55                  60                  65                  70

GGT GAA TTG ACC TCA AAT GGA AGA CTG ACT GCT CAG TTT GGT ATA ACC 334
Gly Glu Leu Thr Ser Asn Gly Arg Leu Thr Ala Gln Phe Gly Ile Thr
                75                  80                  85

AGA AAG GAC AGC TTC CTG AAT ATC TCA GCA TCC ATA CCT AGT GAT GTA 382
Arg Lys Asp Ser Phe Leu Asn Ile Ser Ala Ser Ile Pro Ser Asp Val
            90                  95                  100

GGC ATC TAC TTC TGT GCT 400
Gly Ile Tyr Phe Cys Ala
        105
IV - INFORMATION POUR SEQ ID N° 3
   CARACTERISTIQUES DES SEQUENCES
      TYPE : nucléotide et sa protéine correspondante
      LONGUEUR : 339 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 04
      SEQUENCE Vα w26
   DESCRIPTION DE LA SEQUENCE 
AGCTAAGGG ATG GAG ACT GTT CTG CAA GTA CTC CTA GGG ATA TTG GGG 48
Met Glu Thr Val Leu Gln Val Leu Leu Gly Ile Leu Gly
1               5                   10

TTC CAA GCA GCC TGG GTC AGT AGC CAA GAA CTG GAG CAG AGT CCT CAG 96
Phe Gln Ala Ala Trp Val Ser Ser Gln Glu Leu Glu Gln Ser Pro Gln
    15                  20                  25

TCC TTG ATC GTC CAA GAG GGA AAG AAT CTC ACC ATA AAC TGC ACG TCA 144
Ser Leu Ile Val Gln Glu Gly Lys Asn Leu Thr Ile Asn Cys Thr Ser
30                  35                  40                  45

TCA AAG ACG TTA TAT GGC TTA TAC TGG TAT AAG CAA AAG TAT GGT GAA 192
Ser Lys Thr Leu Tyr Gly Leu Tyr Trp Tyr Lys Gln Lys Tyr Gly Glu
                50                  55                  60

GGT CTT ATC TTC TTG ATG ATG CTA CAG AAA GGT GGG GAA GAG AAA AGT 240
Gly Leu Ile Phe Leu Met Met Leu Gln Lys Gly Gly Glu Glu Lys Ser
            65                  70                  75

CAT GAA AAG ATA ACT GCC AAG TTG GAT GAG AAA AAG CAG CAA AGT TCC 288
His Glu Lys Ile Thr Ala Lys Leu Asp Glu Lys Lys Gln Gln Ser Ser
        80                  85                  90

CTG CAT ATC ACA GCC TCC CAG CCC AGC CAT GCA GGC ATC TAC CTC TGT 336
Leu His Ile Thr Ala Ser Gln Pro Ser His Ala Gly Ile Tyr Leu Cys
    95                  100                 105

GGA 339
Gly
110
V - INFORMATION POUR SEQ ID N° 4
   CARACTERISTIQUES DES SEQUENCES
      TYPE: nucléotide et sa protéine correspondante
      LONGUEUR : 335 paires de bases
      NOMBRE DE BRINS : simple
      CONFIGURATION : linéaire
      TYPE DE MOLECULE : ADNc pour ARNm
   ORIGINE
      ORGANISME :humain
      LIGNEE CELLULAIRE : lymphocytes T humains
   CARACTERISTIQUES
      NOM DE L'ADN : IGR a 05
      SEQUENCE Vα w27
   DESCRIPTION DE LA SEQUENCE 
AGAAAAAAAA AATGAAGAAG CTACTAGCAA TGATCCTGTG GCTTCAACTA 50

GACCGGTTAA GTGGAGAGCT GAAAGTG GAA CAA AAC CCT CTG TTC 95
Glu Gln Asn Pro Leu Phe
1               5

CTG AGC ATG CAG GAG GGA AAA AAC TAT ACC ATC TAC TGC AAT TAT TCA 143
Leu Ser Met Gln Glu Gly Lys Asn Tyr Thr Ile Tyr Cys Asn Tyr Ser
            10                  15                  20

ACC ACT TCA GAC AGA CTG TAT TGG TAC AGG CAG GAT CCT GGG AAA AGT 191
Thr Thr Ser Asp Arg Leu Tyr Trp Tyr Arg Gln Asp Pro Gly Lys Ser
        25                  30                  35

CTG GAA TCT CTG TTT GTG TTG CTA TCA AAT GGA GCA GTG AAG CAG GAG 239
Leu Glu Ser Leu Phe Val Leu Leu Ser Asn Gly Ala Val Lys Gln Glu
    40                  45                  50

GGA CGA TTA ATG GCC TCA CTT GAT ACC AAA GCC CGT CTC AGC ACC CTC 287
Gly Arg Leu Met Ala Ser Leu Asp Thr Lys Ala Arg Leu Ser Thr Leu
55                  60                  65                  70

CAC ATC ACA GCT GCC GTG CAT GAC CTC TCT GCC ACC TAC TTC TGT GCC 335
His Ile Thr Ala Ala Val His Asp Leu Ser Ala Thr Tyr Phe Cys Ala
                75                  80                  85
VI - INFORMATION POUR SEQ ID N° 5
   CARACTERISTIQUES DES SEQUENCES
      TYPE : nucléotide et sa protéine correspondante
      LONGUEUR : 361 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 07
      SEQUENCE Vα w29
   DESCRIPTION DE LA SEQUENCE 
GAAGCTGACT GGATATTCTG GCAGGCCAAG G ATG GAG ACT CTC CTG 46
Met Glu Thr Leu Leu
1               5

AAA GTG CCT TCA GGC ACC TTG TTG TGG CAG TTG ACC TGG GTG GGA AGC 94
Lys Val Pro Ser Gly Thr Leu Leu Trp Gln Leu Thr Trp Val Gly Ser
                10                  15                  20

CAA CAA CCA GTG CAG AGT CCT CAA GCC GTG ATC CTC CGA GAA GGG GAA 142
Gln Gln Pro Val Gln Ser Pro Gln Ala Val Ile Leu Arg Glu Gly Glu
            25                  30                  35

GAT GCT GTC ACC AAC TGC AGT TCC TCC AAG GCT TTA TAT TCT GTA CAC 190
Asp Ala Val Thr Asn Cys Ser Ser Ser Lys Ala Leu Tyr Ser Val His
        40                  45                  50

TGG TAC AGG CAG AAG CAT GGT GAA GCA CCC GTC TTC CTG ATG ATA TTA 238
Trp Tyr Arg Gln Lys His Gly Glu Ala Pro Val Phe Leu Met Ile Leu
    55                  60                  65

CTG AAG GGT GGA GAA CAG ATG CGT CGT GAA AAA ATA TCT GCT TCA TTT 286
Leu Lys Gly Gly Glu Gln Met Arg Arg Glu Lys Ile Ser Ala Ser Phe
70                  75                  80                  85

AAT GAA AAA AAG CAG CAA AGC TCC CTG TAC CTT ACG GCC TCC CAG CTC 334
Asn Glu Lys Lys Gln Gln Ser Ser Leu Tyr Leu Thr Ala Ser Gln Leu
                90                  95                  100

AGT TAC TCA GGA ACC TAC TTC TGC GGG 361
Ser Tyr Ser Gly Thr Tyr Phe Cys Gly
            105                 110
VII - INFORMATION POUR SEQ ID N° 6
   CARACTERISTIQUES DES SEQUENCES
      TYPE : nucléotide et sa protéine correspondante
      LONGUEUR : 569 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 08
      SEQUENCE Vα 1
   DESCRIPTION DE LA SEQUENCE 
TCAGTTTCTT 10
CTTCCTGCAG CTGGTTGAGT TCTTTCCAGA CAAAGACAAG TGACAAGAAT TAGAGGTTTA 70
AAAAGCAACC AGATTCATCT CAGCAGCTTT TGTAGTTTTA AATAAGCAAG GAGTTTCTCC 130
AGCGAAACTT CCTCACACCT CTTGGTCTTG GTCTCTTCAG ACACTTTCCT TCCTGTTCTC 190
TGGAGATCTT GCAGAAAAGA GCCTGCAGTG TTTCCCTTGC TCAGCC ATG CTC CTG 245
Met Leu Leu
1
GAG CTT ATC CCA CTG CTG GGG ATA CAT TTT GTC CTG AGA ACT GCC AGA 293
Glu Leu Ile Pro Leu Leu Gly Ile His Phe Val Leu Arg Thr Ala Arg
    5                   10                  15
GCC CAG TCA GTG ACC CAG CCT GAC ATC CAC ATC ACT GTC TCT GAA GGA 341
Ala Gln Ser Val Thr Gln Pro Asp Ile His Ile Thr Val Ser Glu Gly
20                  25                  30                  35
GCC TCA CTG GAG TTG AGA TGT AAC TAT TCC TAT GGG GCA ACA CCT TAT 389
Ala Ser Leu Glu Leu Arg Cys Asn Tyr Ser Tyr Gly Ala Thr Pro Tyr
                40                  45                  50
CTC TTC TGG TAT GTC CAG TCC CCC GGC CAA GGC CTC CAG CTG CTC CTG 437
Leu Phe Trp Tyr Val Gln Ser Pro Gly Gln Gly Leu Gln Leu Leu Leu
            55                  60                  65
AAG TAC TTT TCA GGA GAC ACT CTG GTT CAA GGC ATT AAA GGC TTT GAG 485
Lys Tyr Phe Ser Gly Asp Thr Leu Val Gln Gly Ile Lys Gly Phe Glu
        70                  75                  80
GCT GAA TTT AAG AGG AGT CAA TCT TCC TTC AAC CTG AGG AAA CCC TCT 533
Ala Glu Phe Lys Arg Ser Gln Ser Ser Phe Asn Leu Arg Lys Pro Ser
    85                  90                  95
GTG CAT TGG AGT GAT GCT GCT GAG TAC TTC TGT GCT 569
Val His T[?]p Ser Asp Ala Ala Glu Tyr Phe Cys Ala
100 105 110
VIII - INFORMATION POUR SEQ ID N° 7
   CARACTERISTIQUES DES SEQUENCES
      TYPE : nucléotide et sa protéine correspondante
      LONGUEUR : 330 paires de bases
      NOMBRE DE BRINS : simple
      CONFIGURATION : linéaire
      TYPE DE MOLECULE : ADNc pour ARNm
      SEQUENCE CONSENSUS
   ORIGINE
      ORGANISME : humain
      LIGNEE CELLULAIRE : lymphocytes T humains
   CARACTERISTIQUES
      NOM DE L'ADN : IGR a 09
      SEQUENCE Vα 2
   DESCRIPTION DE LA SEQUENCE 
AAA TCC TTG AGA GTT TTA CTA GTG ATC CTG TGG CTT CAG CTG AGC CGG 48
Lys Ser Leu Arg Val Leu Leu Val Ile Leu Trp Leu Gln Leu Ser Arg
1               5                   10                  15

GTT TGG AGC CAA CAG AAG GAG GTG GAG CAG AAT TCT GGA CCC CTC AGT 96
Val Trp Ser Gln Gln Lys Glu Val Glu Gln Asn Ser Gly Pro Leu Ser
            20                  25                  30

GTT CCA GAG GGA GCC ATT GCC TCT CTC AAC TGC ACT TAC AGT GAC CGA 144
Val Pro Glu Gly Ala Ile Ala Ser Leu Asn Cys Thr Tyr Ser Asp Arg
        35                  40                  45

GGT TCC CAG TCC TTC TTC TGG TAC AGA CAA TAT TCT GGG AAA AGC CCT 192
Gly Ser Gln Ser Phe Phe Trp Tyr Arg Gln Tyr Ser Gly Lys Ser Pro
    50                  55                  60

GAG TTG ATA ATG TCC ATA TAC TCC AAT GGT GAC AAA GAA GAT GGA AGG 240
Glu Leu Ile Met Ser Ile Tyr Ser Asn Gly Asp Lys Glu Asp Gly Arg
65                  70                  75                  80

TTT ACA GCA CAG CTC AAT AAA GCC AGC CAG TAT GTT T[?]T CTG CTC ATC 288
Phe Thr Ala Gln Leu Asn Lys Ala Ser Gln Tyr Val Ser Leu Leu Ile
                85                  90                  95

AGA GAC TCC CAG CCC AGT GAT TCA GCC ACC TAC CTC TGT GCC 330
Arg Asp Ser Gln Pro Ser Asp Ser Ala Thr Tyr Leu Cys Ala
            100                 105                 110
IX - INFORMATION POUR SEQ ID N° 8
   CARACTERISTIQUES DES SEQUENCES
      TYPE: nucléotide et sa protéine correspondante
      LONGUEUR : 400 paires de bases
      NOMBRE DE BRINS : simple
      CONFIGURATION : linéaire
      TYPE DE MOLECULE : ADNc pour ARNm
   SEQUENCE CONSENSUS
   ORIGINE
      ORGANISME : humain
      LIGNEE CELLULAIRE : lymphocytes T humains
   CARACTERISTIQUES
      NOM DE L'ADN : IGR a 10
      SEQUENCE Vα 5
   DESCRIPTION DE LA SEQUENCE 
GCCAAACAGA ATGGCTTTTT GGCTGAGAAG GCTGGGTCTA CATTTCAGGC 50

CACATTTGGG GAGACGA ATG GAG TCA TCC CTG GGA GGT GTT TTG 94
Met Glu Ser Ser Leu Gly Gly Val Leu
1               5

CTG ATT TTG TGG CTT CAA GTG GAC TGG GTG AAG AGC CAA AAG ATA GAA 142
Leu Ile Leu Trp Leu Gln Val Asp Trp Val Lys Ser Gln Lys Ile Glu
10                  15                  20                  25

CAG AAT TCC GAG GCC CTG AAC ATT CAG GAG GGT AAA ACG GCC ACC CTG 190
Gln Asn Ser Glu Ala Leu Asn Ile Gln Glu Gly Lys Thr Ala Thr Leu
                30                  35                  40

ACC TGC AAC TAT ACA AAC TAT TCT CCA GCA TAC TTA CAG TGG TAC CGA 238
Thr Cys Asn Tyr Th- Asn Tyr Ser Pro Ala Tyr Leu Gln Trp Tyr Arg
45 50 55

CAA GAT CCA GGA AGA GGC CCT GTT TTC TTG CTA CTC ATA CGT GAA AAT 286
Gln Asp Pro Gly Arg Gly Pro Val Phe Leu Leu Leu Ile Arg Glu Asn
60 65 70

GAG AAA GAA AAA AGG AAA GAA AGA CTG AAG GTC ACC TTT GAT ACC ACC 334
Glu Lys Glu Lys Arg Lys Glu Arg Leu Lys Val Thr Phe Asp Thr Thr
75 80 85

CTT AAA CAG AGT TTG TTT CAT ATC ACA GCC TCC CAG CCT GCA GAC TCA 382
Leu Lys Gln Ser Leu Phe His Ile Thr Ala Ser Gln Pro Ala Asp Ser
90 95 100 105

GCT ACC TAC CTC TGT GCT 400
Ala Thr Tyr Leu Cys Ala
110
X - INFORMATION POUR SEQ ID N° 9
   CARACTERISTIQUES DES SEQUENCES
      TYPE : nucléotide et sa protéine correspondante
      LONGUEUR : 386 paires de bases
      NOMBRE DE BRINS : simple
      CONFIGURATION : linéaire
      TYPE DE MOLECULE : ADNc pour ARNm
   SEQUENCE CONSENSUS
   ORIGINE
      ORGANISME : humain
      LIGNEE CELLULAIRE : lymphocytes T humains
   CARACTERISTIQUES
      NOM DE L'ADN : IGR a 11
      SEQUENCE Vα 7
   DESCRIPTION DE LA SEQUENCE 
GCCTTCTGCA GACTCCAATG GCTCAGGAAC TGGGAATGCA GTGCCAGGCT 50

CGTGGTATCC TGCAGCAG ATG TGG GGA GTT TTC CTT CTT TAT GTT 95
Met Trp Gly Val Phe Leu Leu Tyr Val
1               5

TCC ATG AAG ATG GGA GGC ACT ACA GGA CAA AAC ATT GAC CAG CCC ACT 143
Ser Met Lys Met Gly Gly Thr Thr Gly Gln Asn Ile Asp Gln Pro Thr
10                  15                  20                  25

GAG ATG ACA GCT ACG GAA GGT GCC ATT GTC CAG ATC AAC TGC ACG TAC 191
Glu Met Thr Ala Thr Glu Gly Ala Ile Val Gln Ile Asn Cys Thr Tyr
                30                  35                  40

CAG ACA TCT GGG TTC AAC GGG CTG TTC TGG TAC CAG CAA CAT GCT GGC 239
Gln Thr Ser Gly Phe Asn Gly Leu Phe Trp Tyr Gln Gln His Ala Gly
            45                  50                  55

GAA GCA CCC ACA TTT CTG TCT TAC AAT GTT CTG GAT GGT TTG GAG GAG 287
Glu Ala Pro Thr Phe Leu Ser Tyr Asn Val Leu Asp Gly Leu Glu Glu
        60                  65                  70

AAA GGT CGT TTT TCT TCA TTC CTT AGT CGG TCT AAA GGG TAC AGT TAC 335
Lys Gly Arg Phe Ser Ser Phe Leu Ser Arg Ser Lys Gly Tyr Ser Tyr
    75                  80                  85

CTC CTT TTG AAG GAG CTC CAG ATG AAA GAC TCT GCC TCT TAC CTC TGT 383
Leu Leu Leu Lys Glu Leu Gln Met Lys Asp Ser Ala Ser Tyr Leu Cys
90                  95                  100                 105

GCT 386
Ala
XI - INFORMATION POUR SEQ ID N° 10
   CARACTERISTIQUES DES SEQUENCES
      TYPE: nucléotide et sa protéine correspondante
      LONGUEUR : 383 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 12
      SEQUENCE Vα 22
   DESCRIPTION DE LA SEQUENCE 
TGTGACTTCT TCATGTTAAG GATCAAGACC ATTATTTGGG TAACACACTA 50

AAG ATG AAC TAT TCT CCA GGC TTA GTA TCT CTG ATA CTC TTA CTG 95
Met Asn Tyr Ser Pro Gly Leu Val Ser Leu Ile Leu Leu Leu
1               5                   10

CTT GGA AGA ACC CGT GGA GAT TCA GTG ACC CAG ATG GAA GGG CCA GTG 143
Leu Gly Arg Thr Arg Gly Asp Ser Val Thr Gln Met Glu Gly Pro Val
15                  20                  25                  30

ACT CTC TCA GAA GAG GCC TTC CTG ACT ATA AAC TGC ACG TAC ACA GCC 191
Thr Leu Ser Glu Glu Ala Phe Leu Thr Ile Asn Cys Thr Tyr Thr Ala
                35                  40                  45

ACA GGA TAC CCT TCC CTT TTC TGG TAT GTC CAA TAT CCT GGA GAA GGT 239
Thr Gly Tyr Pro Ser Leu Phe Trp Tyr Val Gln Tyr Pro Gly Glu Gly
            50                  55                  60

CTA CAG CTC CTC CTG AAA GCC ACG AAG GCT GAT GAC AAG GGA AGC AAC 287
Leu Gln Leu Leu Leu Lys Ala Thr Lys Ala Asp Asp Lys Gly Ser Asn
        65                  70                  75

AAA GGT TTT GAA GCC ACA TAC CGT AAA GAA ACC ACT TCT TTC CAC TTG 335
Lys Gly Phe Glu Ala Thr Tyr Arg Lys Glu Thr Thr Ser Phe His Leu
    80                  85                  90

GAG AAA GGC TCA GTT CAA GTG TCA GAC TCA GCG GTG TAC TTC TGT GCT 383
Glu Lys Gly Ser Val Gln Val Ser Asp Ser Ala Val Tyr Phe Cys Ala
95                  100                 105                 110
XII - INFORMATION POUR SEQ ID N° 11
   CARACTERISTIQUES DES SEQUENCES
      TYPE : nucléotide et sa protéine correspondante
      LONGUEUR : 364 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : IGR a 13
      SEQUENCE Vα 16
   DESCRIPTION DE LA SEQUENCE 
AATCCCGCCC GCCGTGAGCT TAGCTGGAGC C ATG GCC TCT GCA CCC 46
Met Ala Ser Ala Pro
1               5

ATC TCG ATG CTT GCG ATG CTC TTC ACA TTG AGT GGG CTG AGA GCT CAG 94
Ile Ser Met Leu Ala Met Leu Phe Thr Leu Ser Gly Leu Arg Ala Gln
                10                  15                  20

TCA GTG GCT CAG CCG GAA GAT CAG GTC AAC GTT GCT GAA GGG AAT CCT 142
Ser Val Ala Gln Pro Glu Asp Gln Val Asn Val Ala Glu Gly Asn Pro
            25                  30                  35

CTG ACT GTG AAA TGC ACC TAT TCA GTC TCT GGA AAC CCT TAT CTT TTT 190
Leu Thr Val Lys Cys Thr Tyr Ser Val Ser Gly Asn Pro Tyr Leu Phe
        40                  45                  50

TGG TAT GTT CAA TAC CCC AAC CGA GGC CTC CAG TTC CTT CTG AAA TAC 238
Trp Tyr Val Gln Tyr Pro Asn Arg Gly Leu Gln Phe Leu Leu Lys Tyr
    55                  60                  65

ATC ACA GGG GAT AAC CTG GTT AAA GGC AGC TAT GGC TTT GAA GCT GAA 286
Ile Thr Gly Asp Asn Leu Val Lys Gly Ser Tyr Gly Phe Glu Ala Glu
70                  75                  80                  85

TTT AAC AAG AGC CAA ACC TCC TTC CAC CTG AAG AAA CCA TCT GCC CTT 334
Phe Asn Lys Ser Gln Thr Ser Phe His Leu Lys Lys Pro Ser Ala Leu
                90                  95                  100

GTG AGC GAC TCC GCT TTG TAC TTC TGT GCT 364
Val Ser Asp Ser Ala Leu Tyr Phe Cys Ala
            105                 110
XIII - INFORMATION POUR SEQ ID N° 12
   CARACTERISTIQUES DES SEQUENCES
      TYPE : nucléotide et sa protéine correspondante
      LONGUEUR : 264 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 01
      SEQUENCE Jα
   DESCRIPTION DE LA SEQUENCE 
CCTTCAAGGA AAATTAAGGC AAATAGAATT GGGCTGGGGA GTTGCTACTT ATTAGTATTC 60

CTCCCACGTT CTAACCTAAT TATAAGGAGG TTGTTTTGGC CATGGGCAGT CATCTCAGGT 120

TTTGTTTTCC TGCTTTCCTC CCTAACCTCC ACCTGTCTTC CTAGAGGCCT GAGTCAAGGT 180

TATTGCAATA GCACTAAAGA CTGTGT AAC ACC AAT GCA GGC AAA TCA ACC TTT 233
Asn Thr Asn Ala Gly Lys Ser Thr Phe
1               5

GGG GAT GGG ACT ACG CTC ACT GTG AAG CCA 264
Gly Asp Gly Thr Thr Leu Thr Val Lys Pro
10                  15
XIV - INFORMATION POUR SEQ ID N° 13
   CARACTERISTIQUES DES SEQUENCES
      TYPE : nucléotide et sa protéine correspondante
      LONGUEUR : 277 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 02
      SEQUENCE Jα
   DESCRIPTION DE LA SEQUENCE 
AAGGACACAG ACTGCCTGCA TGAAGGCTGG AGCTGGGCCC AGGATGAGGA AAGGCCTCAG 60

GAAGGAAGGG CTGACACGAA ATAAGGAATA CCATGGCATT CATGAGATGT GCGTCTGAAT 120

CCTCTCTCTT GCCTGAGAAG CTTTAGCTTC CACCTTGAGA CACAAAACAT GTGGTTATGA 180

AGAGATGACA AGGTTTTTGT AAAAGAATGA GCCATTGTGG ATA GGC TTT GGG AAT 234
Ile Gly Phe Gly Asn
1               5

GTG CTG CAT TGC GGG TCC GGC ACT CAA GTG ATT GTT TTA CCA 277
Val Leu His Cys Gly Ser Gly Thr Gln Val Ile Val Leu Pro
                10                  15
XVI - INFORMATION POUR SEQ ID N° 15
   CARACTERISTIQUES DES SEQUENCES
      TYPE : nucléotide et sa protéine correspondante
      LONGUEUR : 60 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 04
      SEQUENCE Jα
   DESCRIPTION DE LA SEQUENCE 
TA GAT ACT GGA GGC TTC AAA ACT ATC TTT GGA GCA GGA ACA AGA CTA 47
Asp Thr Gly Gly Phe Lys Thr Ile Phe Gly Ala Gly Thr Arg Leu
1               5                   10                  15

TTT GTT AAA GCA A 60
Phe Val Lys Ala
XVII - INFORMATION POUR SEQ ID N° 16
   CARACTERISTIQUES DES SEQUENCES
      TYPE : nucléotide et sa protéine correspondante
      LONGUEUR : 59 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 05
      SEQUENCE Jα
   DESCRIPTION DE LA SEQUENCE 
C CTA ACT GGG GCA AAC AAC GTC TTC TTT GGG ACT GGA ACG AGA CTC 46
Leu Thr Gly Ala Asn Asn Val Phe Phe Gly Thr Gly Thr Arg Leu
1               5                   10                  15

ACC GTT CTT CCC T 59
Thr Val Leu Pro
XVIII - INFORMATION POUR SEQ ID N° 17
   CARACTERISTIQUES DES SEQUENCES
      TYPE : nucléotide et sa protéine correspondante
      LONGUEUR : 60 paires de bases
      NOMBRE DE BRINS : simple
      CONFIGURATION : linéaire
      TYPE DE MOLECULE : ADNc pour ARNm
   ORIGINE
      ORGANISME :humain
      LIGNEE CELLULAIRE : lymphocytes T humains
   CARACTERISTIQUES
      NOM DE L'ADN : Ja 06
      SEQUENCE Jα
   DESCRIPTION DE LA SEQUENCE 
AT GGA GGA AGC CAA GGA AAT CTC ATC TTT GGA AAA GGC ACT AAA CTC 47
Gly Gly Ser Gln Gly Asn Leu Ile Phe Gly Lys Gly Thr Lys Leu
1               5                   10                  15

TCT GTT AAA CCA A 60
Ser Val Lys Pro
XIX - INFORMATION POUR SEQ ID N° 18
   CARACTERISTIQUES DES SEQUENCES
      TYPE : nucléotide et sa protéine correspondante
      LONGUEUR : 56 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 07
      SEQUENCE Jα
   DESCRIPTION DE LA SEQUENCE 
GGA GCC AAT AGT AAG CTG ACA TTT GGA AAA GGA ATA ACT CTG AGT GTT 48
Gly Ala Asn Ser Lys Leu Thr Phe Gly Lys Gly Ile Thr Leu Ser Val
1               5                   10                  15

AGA CCA GA 56
Arg Pro
XX - INFORMATION POUR SEQ ID N° 19
   CARACTERISTIQUES DES SEQUENCES
      TYPE: nucléotide et sa protéine correspondante
      LONGUEUR : 57 paires de bases
      NOMBRE DE BRINS : simple
      CONFIGURATION : linéaire
      TYPE DE MOLECULE : ADNc pour ARNm
   ORIGINE
      ORGANISME : humain
      LIGNEE CELLULAIRE : lymphocytes T humains
   CARACTERISTIQUES
      NOM DE L'ADN : Ja 08
      SEQUENCE Jα
   DESCRIPTION DE LA SEQUENCE 
CT GGT GGC TAC AAT AAG CTG ATT TTT GGA GCA GGG ACC AGG CTG GCT 47
Gly Gly Tyr Asn Lys Leu Ile Phe Gly Ala Gly Thr Arg Leu Ala
1               5                   10                  15

GTA CAC CCA T 57
Val His Pro
XXI - INFORMATION POUR SEQ ID N° 20
   CARACTERISTIQUES DES SEQUENCES
      TYPE : nucléotide et sa protéine correspondante
      LONGUEUR : 50 paires de bases
      NOMBRE DE BRINS : simple
      CONFIGURATION : linéaire
      TYPE DE MOLECULES : ADNc pour ARNm
   ORIGINE
      ORGANISME : humain
      LIGNEE CELLULAIRE : lymphocytes T humains
   CARACTERISTIQUES
      NOM DE L'ADN : Ja 09
      SEQUENCE Jα
   DESCRIPTION DE LA SEQUENCE

T GGA AAC AAG CTG GTC TTT GGC GCA GGA ACC ATT CTG AGA GTC AAG 46
Gly Asn Lys Leu Val Phe Gly Ala Gly Thr Ile Leu Arg Val Lys
1               5                   10                  15

TCC T 50
Ser

XXII - INFORMATION POUR SEQ ID N° 21 à 25
TYPE OLIGONUCLEOTIDES
DESCRIPTION DES SEQUENCES

| | |
|---|---|
| N° 21 A | (5'-GTTGCTCCAGGCCACAGCACTG) |
| N° 22 poly C | (5'-GCATGCGCGCGGCCGCGGAGG-14C) |
| N° 23 B | (5'-GTCCATAGACCTCATGTCCAGCACAG) |
| N° 24 C | (5'-ATACACATCAGAATTCTTACTTTG) |
| N° 25 D | (5'-GTCACTGGATTTAGAGTCT) |

XXIII - INFORMATION POUR SEQ ID N° 26 à 63
TYPE OLIGONUCLEOTIDES
DESCRIPTION DES SEQUENCES

| SEQ ID N° | Sequence | Type | Clone | Position |
|---|---|---|---|---|
| 26 | 5'-GGCATTAACGGTTTTGAGGCTGGA-3' | Vα1 | **AB22** | 235 |
| 27 | 5'-CAGTGTTCCAGAGGGAGCCATTGC-3' | Vα2 | IGRa09 | **93**^{*****} |
| 20 | 5'-CCCGGCAGCAGACACTGCTTCTTA-3' | Vα3 | HAP05 | 297 |
| 29 | 5'-TTGGTATCGACAGCTTCCCTCCCA-3' | Vα4 | HAP08 | 153 |
| 30 | 5'-CGGCCACCCTGACCTGCAACTATA-3' | Vα5 | IGRa10 | **113** |
| 31 | 5'-TCCGCCAACCTTGTCATCTCCGCT-3 | Vα6 | HAP01 | 287 |
| 32 | 5'-GCAACATGCTGGCGGAGCACCCAC-3 | Vα7 | IGRa11 | 159 |
| 33 | 5'-CATTCGTTCAAATGTGGGCAAAAG-3 | Vα8 | HAP41 | 204 |
| 34 | 5'-CCAGTACTCCAGACAACGCCTGCA-3 | Vα9 | HAVP36 | 168 |
| 35 | 5'-CACTGCGGCCCAGCCTGGTGATAC-3 | Vα10 | **HAP58** | 282 |
| 36 | 5 ' -CGCTGCTCATCCTCCAGGTGCGGG-3' | Vα11 | AB19 | 254* |
| 37 | 5' -TCGTCGGAACTCTTTTGATGAGCA-3' | Vα12 | V12MA483 | 213 |
| 38 | 5'-TTCATCAAAACCCTTGGGGACAGC-3' | Vα13 | HAVT15 | 152^{*} |
| 39 | 5'-CCCAGCAGGCAGATGATTCTCGTT-3' | Vα14 | HAVT20 | 181 |
| 40 | 5'-TTGCAGACACCGAGACTGGGGACT-3' | Vα15 | HAVT31 | 278 |
| 41 | 5'-TCAACGTTGCTGAAGGGAATCCTC-3' | Vα16 | IGRa13 | 89 |
| 42 | 5' -TGGGAAAGGCCGTGCATTATTGAT-3' | Vα17 | AB11 | 204 |
| 43 | 5'-CAGCACCAATTTCACCTGCAGCTT-3' | Vα18 | AB21 | 114 |
| 44 | 5'-ACACTGGCTGCAACAGCATCCAGG-3' | Vα19 | AC24 | 162 |
| 45 | 5'-TCCCTGTTTATCCCTGCCGACAGA-3' | Vα20 | AE212 | 232 |
| 46 | 5'-AGCAAAATTCACCATCCCTGAGCG-3' | Vα21 | AF211 | 92 |
| 47 | 5'-CCTGAAAGCCACGAAGGCTGATGA-3' | Vα22 | IGRa12 | 197 |
| 48 | 5'-TGCCTCGCTGGATAAATCATCAGG-3' | Vαw23 | IGRa01 | 246 |
| 49 | 5'-CTGGATGCAGACACAAAGCAGAGC-3' | Vαw24 | IGRa02 | 259 |
| 50 | 5'-TGGCThCGGTACAAGCCGGACCCT-3' | Vαw25 | IGRa03 | 148 |
| 51 | 5'-AGCGCAGCCATGCAGGCATGTACC-3' | Vαw26 | IGRa04 | 299 |
| 52 | 5'-AAGCCCGTCTCAGCACCCTCCACA-3' | Vαw27 | IGRa05 | 268* |
| 53 | 5'-TGGTTGTGCACGAGCGACAGACTG-3 | Vαw28 | IGRa06 | 95 |
| 54 | 5'-GAAGGGTGGAGAACAGATGCGTCG-3' | Vαw29 | IGRa07 | 210 |
| 55 | 5'-ATACACATCAGAATTCTTACTTTG-3' | CαA | | 129 |
| 56 | 5'-GTTGCTCCAGGCCGCGGCACTGTT-3' | CαB | | 201 |
| 57 | 5'-GTCACTGGATTTAGAGTCT-3' | CαC | | 57 |
| 58 | 5'-ATTTGCGGTGGACGATGGAGGGGC-3' | Act 1 | B-actine | 1161 |
| 59 | 5'-GGCATCGTCACCAACTGGGACGAC-3' | Act 2 | B-actine | 261 |
| 60 | 5'-ACCACCACGGCGGAGCGGG-3' | Act 3 | B-actine | 642 |
| 61 | 5'-CGGGCTGCTCCTTGAGGGGCTGCG-3' | CβF | | 135 |
| 62 | 5'-CCCACCCGAGGTCGCTGTG-3' | CβK | | 20 |
| 63 | 5'-TCTGCTTCTGATGGCTCAA-3' | CβC | | 58 |

## Revendications

1. Séquence nucléotidique codant pour une région variable de chaîne, α des récepteurs des lymphocytes T humains, correspondant à un ADNc comprenant une séquence nucléotidique correspondant à la séquence SEQ ID N°1.

2. Séquence nucléotidique codant pour une région variable de chaîne α des récepteurs des lymphocytes T humains correspondant à la séquence 1 à 200 de SEQ ID N° 1.

3. Peptides codés par les séquences nucléotidiques telles que définies à l'une des revendications 1 à 2.

4. Vecteurs d'expression comprenant une séquence d'ADN codant pour l'un des peptides tel que défini à la revendication 3.

5. Hôtes non-humains transformés avec un vecteur selon la revendication 4.

6. Anticorps dirigés contre un déterminant antigénique d'un des peptides définis à la revendication 3.

7. Anticorps selon la revendication 6 qui est un anticorps monoclonal.

8. Fragment Fab, Fab' ou F(ab')2 d'un anticorps monoclonal selon la revendication 7.

9. Anticorps monoclonal ou fragment de celui-ci selon la revendication 7 ou 8 auxquels sont liés un marqueur détectable et/ou au moins une molécule thérapeutique.

10. Hybridomes produisant un anticorps selon la revendication 7.

11. Composition de diagnostic comprenant un ou plusieurs anticorps monoclonaux ou fragments de ceux-ci tels que définis à l'une quelconque des revendications 7 à 9.

12. Composition thérapeutique comprenant un ou plusieurs anticorps monoclonaux ou fragments de ceux-ci tels que définis à l'une quelconque des revendications 7 à 9.

13. Composition selon la revendication 12 comprenant des dérivés contenant une molécule cytotoxique ou un radioisotope.

14. Composition thérapeutique comprenant au moins l'un des peptides définis à la revendication 3.

15. Composition thérapeutique comprenant des oligonucléotides antisens correspondant à la séquence d'un segment Vα telle que définie à la revendication 2.

16. Utilisation, en tant qu'amorces pour l'amplification d'ADN, de séquences nucléotidiques d'environ au moins 17 nucléotides comprises dans la séquence d'un segment Vα telle; que définie, à la revendication 2.

17. Utilisation, en tant que sonde de détection, de séquences nucléotidiques d'environ au moins 10 nucléotides comprises dans la séquence d'un segment Vα telle que définie à la revendication 2.

18. Oligonucléotide, utilisable comme amorce pour l'amplification d'ADN correspondant à une région variable de chaîne α des récepteurs de cellules T, de séquence SEQ ID N° 49.

19. Utilisation, comme amorce pour l'amplification d'ADN correspondant à une région variable de chaînes α des récepteurs de cellules T, d'oligonucléotide selon la revendication 18.

20. Procédé de détection de séquence nucléotidique codant pour un segment Vα des récepteurs T ou d'ADNc correspondant au produit de transcription de celle-ci, dans un échantillon biologique, **caractérisé en ce qu'**il comprend :
a) l'amplification de l'ADN avec au moins une paire d'amorces formée par l'oligonucléotide selon la revendication 18 et un oligonucléotide appartenant au segment Cα, et
b) la détection des séquences amplifiées avec une sonde Cα.

21. Procédé selon la revendication 20, **caractérisé en ce que** l'on effectue l'amplification en présence d'une paire d'amorces contrôle et la détection à l'aide d'une sonde contrôle.

22. Kit de diagnostic pour la mise en oeuvre d'un procédé selon la revendication 20, **caractérisé en ce qu'**il comprend :
a) au moins un oligonucléotide selon la revendication 18,
b) une amorce Cα,
c) une sonde Cα.

23. Kit de diagnostic pour la mise en oeuvre d'un procédé selon la revendication 21, **caractérisé en ce qu'**il comprend :
a) au moins un oligonucléotide selon la revendication 18,
b) une amorce Cα,
c) une paire d'amorce contrôle,
d) une sonde Cα,
e) une sonde contrôle.

## Claims

1. Nucleotide sequence coding for an α-chain variable region in human T-lymphocyte receptors, corresponding to a cDNA comprising a nucleotide sequence corresponding to the sequence SEQ ID No. 1.

2. Nucleotide sequence coding for an α-chain variable region in human T-lymphocyte receptors, corresponding to the sequence 1 to 200 of SEQ ID No. 1.

3. Peptides encoded by the nucleotide sequences as defined in one of Claims 1 to 2.

4. Expression vectors comprising a DNA sequence coding for one of the peptides as defined in Claim 3.

5. Nonhuman hosts, transformed with a vector according to Claim 4.

6. Antibodies against an antigenic determinant of one of the peptides defined in Claim 3.

7. Antibody according to Claim 6, which is a monoclonal antibody.

8. Fab, Fab' or F(ab')2 fragment of a monoclonal antibody according to Claim 7.

9. Monoclonal antibody or fragment thereof according to Claim 7 or 8, to which a detectable marker and/or at least one therapeutic molecule are linked.

10. Hybridomas producing an antibody according to Claim 7.

11. Diagnostic composition comprising one or more monoclonal antibodies or fragments thereof as defined in any of Claims 7 to 9.

12. Therapeutic composition comprising one or more monoclonal antibodies or fragments thereof as defined in any of Claims 7 to 9.

13. Composition according to Claim 12, comprising derivatives which contain a cytotoxic molecule or a radioisotope.

14. Therapeutic composition comprising at least one of the peptides defined in Claim 3.

15. Therapeutic composition comprising antisense-oligonucleotides corresponding to the sequence of a Vα segment as defined in Claim 2.

16. Use of nucleotide sequences of about at least 17 nucleotides present in the sequence of a Vα segment as defined in Claim 2 as primers for the amplification of DNA.

17. Use of nucleotide sequences of about at least 10 nucleotides present in the sequence of a Vα segment as defined in Claim 2 as detection probe.

18. Oligonucleotide which can be used as primer for the amplification of DNA corresponding to an α-chain variable region in T-cell-receptors, of the sequence SEQ ID No. 49.

19. Use of the oligonucleotide according to Claim 18 as primer for the amplification of DNA corresponding to an α-chain variable region in T-cell receptors.

20. Method of detecting, in a biological sample, a nucleotide sequence coding for a Vα segment of the T receptors or for detecting cDNA corresponding to its transcription product, **characterized in that** it comprises:
a) amplification of the DNA with at least one primer pair formed by the oligonucleotide according to Claim 18 and an oligonucleotide which belongs to the Cα segment, and
b) detection of the amplified sequences using a Cα probe.

21. Method according to Claim 20, **characterized in that** the amplification is accomplished in the presence of a control primer pair and detection with the aid of a control probe.

22. Diagnostic kit for carrying out a method according to Claim 20, **characterized in that** it comprises:
a) at least one oligonucleotide according to Claim 18,
b) a Cα primer,
c) a Cα probe.

23. Diagnostic kit for carrying out a method according to Claim 21, **characterized in that** it comprises:
a) at least one oligonucleotide according to Claim 18,
b) a Cα primer,
c) a control primer pair,
d) a Cα probe,
e) a control probe.

## Patentansprüche

1. Nukleotidsequenz, die für eine variable Region der alpha-Kette menschlicher T-Zellen-Rezeptoren kodiert und die einer cDNA entspricht, die eine Nukleotidsequenz gemäß SEQ ID Nr. 1 umfaßt.

2. Nukleotidsequenz, die für eine variable Region der alpha-Kette menschlicher T-Zellen-Rezeptoren kodiert und die der Sequenz 1 bis 200 der SEQ ID Nr. 1 entspricht.

3. Peptide, die von den Nukleotidsequenzen nach einem der Ansprüche 1 bis 2 kodiert werden.

4. Expressionsvektoren, die eine DNA-Sequenz, die für eines der Peptide nach Anspruch 3 kodiert, enthalten.

5. Nichtmenschliche Wirte, die mit einem Vektor nach Anspruch 4 transformiert sind.

6. Antikörper gegen die eine antigene Determinante von einem der Peptide nach Anspruch 3.

7. Antikörper nach Anspruch 6, wobei es sich um einen monoklonalen Antikörper handelt.

8. Fab-, Fab'- oder F(ab')2-Fragment eines monoklonalen Antikörpers nach Anspruch 7.

9. Monoklonaler Antikörper oder Fragment davon nach Anspruch 7 oder 8, an die ein nachweisbarer Marker und/oder mindestens ein therapeutisches Molekül gebunden sind.

10. Hybridome, die einen Antikörper nach Anspruch 7 produzieren.

11. Diagnostische Zusammensetzung, die einen oder mehrere monoklonale Antikörper oder deren Fragmente nach einem der Ansprüche 7 bis 9 umfassen.

12. Diagnostische Zusammensetzung, die einen oder mehrere monoklonale Antikörper oder deren Fragmente nach einem der Ansprüche 7 bis 9 umfassen.

13. Zusammensetzung nach Anspruch 12, die Derivate umfaßt, die ein cytotoxisches Molekül oder ein Radioisotop enthalten.

14. Therapeutische Zusammensetzung, die mindestens eines der Peptide nach Anspruch 3 umfaßt.

15. Therapeutische Zusammensetzung, die Antisense-Oligonukleotide umfaßt, die der Sequenz eines Vα-Abschnitts nach Anspruch 2 entsprechen.

16. Verwendung von Nukleotidsequenzen mit ungefähr mindestens 17 Nukleotiden, die von der Sequenz eines Vα-Abschnitts gemäß Anspruch 2 umfaßt werden, als Primer für die Amplifikation von DNA.

17. Verwendung von Nukleotidsequenzen mit ungefähr mindestens 10 Nukleotiden, die von der Sequenz eines Vα-Abschnitts gemäß Anspruch 2 umfaßt werden, als Nachweissonde.

18. Oligonukleotid, das als Primer für die Amplifikation von DNA, die einer variablen Region der alpha-Kette von T-Zellen-Rezeptoren entspricht, verwendet werden kann, und das der Sequenz SEQ ID Nr. 49 entspricht.

19. Verwendung des Oligonukleotids nach Anspruch 18 als Primer für die Amplifikation von DNA, die einer variablen Region der alpha-Ketten des T-Zellen-Rezeptors entspricht.

20. Verfahren zum Nachweis einer Nukleotidsequenz, die für einen Vα-Abschnitt des T-Rezeptors kodiert, oder von cDNA, die dessen Transkriptionsprodukt entspricht, in einer biologischen Probe, **dadurch gekennzeichnet, daß** es die folgenden Schritte umfaßt:
a) Amplifikation der DNA mit mindestens einem Primer-Paar, das von dem Oligonukleotid nach Anspruch 18 und einem Oligonukleotid, das zu dem Cα-Abschnitt gehört, gebildet wird, sowie
b) Nachweisen der amplifizierten Sequenzen mit einer Cα-Sonde.

21. Verfahren nach Anspruch 20, **dadurch gekennzeichnet, daß** man die Amplifikation in Gegenwart eines Kontroll-Primer-Paars und den Nachweis mit Hilfe einer Kontrollsonde durchführt.

22. Diagnose-Kit zur Durchführung eines Verfahrens nach Anspruch 20, **dadurch gekennzeichnet, daß** es
a) mindestens ein Oligonukleotid nach Anspruch 18,
b) einen Cα-Primer,
c) eine Cα-Sonde
umfaßt.

23. Diagnose-Kit zur Durchführung eines Verfahrens nach Anspruch 21, **dadurch gekennzeichnet, daß** es
a) mindestens ein Oligonukleotid nach Anspruch 18,
b) einen Cα-Primer,
c) ein Kontroll-Primer-Paar,
d) eine Cα-Sonde
e) eine Kontrollsonde
umfaßt.
